(19) Europäisches Patentamt
European Patent Office
Office européen des brevets

(11) EP 0 978 279 A1

# (12) EUROPEAN PATENT APPLICATION

(43) Date of publication:
**09.02.2000 Bulletin 2000/06**

(21) Application number: **99306047.4**

(22) Date of filing: **29.07.1999**

(51) Int Cl.[7]: **A61K 31/404**, C07D 209/42, C12Q 1/48, A61P 5/50, A61P 9/10

(84) Designated Contracting States:
**AT BE CH CY DE DK ES FI FR GB GR IE IT LI LU MC NL PT SE**
Designated Extension States:
**AL LT LV MK RO SI**

(30) Priority: **07.08.1998 US 95790 P**

(71) Applicant: **Pfizer Products Inc.**
**Groton, CT 06340-5146 (US)**

(72) Inventors:
- **Rath, Virginia Leigh**
  **Groton, Connecticut 06340 (US)**
- **Hoover, Dennis Jay**
  **Groton, Connecticut 06340 (US)**
- **Ammirati, Mark**
  **Groton, Connecticut 06340 (US)**

(74) Representative:
**Simpson, Alison Elizabeth Fraser et al**
**Urquhart-Dykes & Lord,**
**91 Wimpole Street**
**London W1M 8AH (GB)**

## (54) Inhibitors of human glycogen phosphorylase

(57) The present invention is directed to a novel binding site for a glycogen phosphorylase inhibitor found within a glycogen phosphorylase enzyme. The novel binding site allows the design novel of glycogen phosphorylase inhibitors.



Printed by Jouve, 75001 PARIS (FR)

## Description

### Background of the Invention

The present invention relates to a method of inhibiting glycogen phosphorylase (GP) by administering any compound with certain structural, physical and spatial characteristics that allow for the interaction of said compound with specific residues of the enzyme. The present invention also relates to a novel crystalline form of glycogen phosphorylase, of a novel binding site for inhibitors of GP and methods enabling the design and selection of inhibitors which bind to the novel binding site.

In spite of the early discovery of insulin and its subsequent widespread use in the treatment of diabetes, and the later discovery of and use of sulfonylureas (e.g. chlorpropamide, tolbutamide, acetohexamide, tolazamide) and biguanides (e.g. phenformin, metformin) alpha-glucosidase inhibitors (e.g. glucophage) and insulin sensitizing agents (e.g. Rezulin) as oral hypolglycemic agents, the need for improvement in treatment of diabetes remains. The use of insulin, necessary in about 10% of diabetic patients in which synthetic hypoglycemic agents are not effective (Type 1 diabetes, insulin dependent diabetes melitus), requires multiple daily doses, usually by self injection. Determination of the proper dosage of insulin requires frequent estimations of the sugar in urine or blood. The administration of an excess dose of insulin causes hypoglycemia, with effects ranging from mild abnormalities in blood glucose to coma, or even death. Treatment of non-insulin dependent diabetes mellitus (Type 2 diabetes, NIDDM) usually consists of a combination of diet, exercise, oral agents, e.g. sulfonylureas, and in more severe cases, insulin. However, the clinically available hypoglycemics are unfortunately fraught with other undesirable manifestations which limit their use. In any event, where one of these agents may fail in an individual case, another may succeed. A continuing need for hypoglycemic agents, which may be safer or succeed where others fail, is clearly evident.

Hepatic glucose production is an important potential target for Type 2 diabetes therapy. The liver is the major regulator of plasma glucose levels in the post absorptive (fasted) state, and the rate of hepatic glucose production in Type 2 diabetes patients is significantly elevated relative to normal individuals. Likewise, in the postprandial (fed) state, where the liver has a proportionately smaller role in the total plasma glucose supply, hepatic glucose production is abnormally high in Type 2 diabetic patients [Gerich, J.E. Horm. Met. Res. 1992, 26, 18].

Glycogenolysis is an important target for interruption of hepatic glucose production. The liver produces glucose by glycogenolysis (breakdown of the glucose polymer glycogen) and gluconeogenesis (synthesis of glucose from 2-and 3-carbon precursors). Several lines of evidence suggest that glycogenolysis may make an important contribution to hepatic glucose output in Type 2 diabetes. First, in normal post absorptive man, 75% of hepatic glucose production is estimated to result from glycogenolysis [Barret, E.J. and Liu, Z. Clin. Endocrin. Metab. 1993, 7, 875]. Second, patients having liver glycogen storage diseases, including Hers' disease (glycogen phosphorylase deficiency), display episodic hypoglycemia [Gorin, F.A. et al. J. Neurogenetics 1987, 4, 293]. These observations suggest that glycogenolysis may be a significant process for hepatic glucose production.

Glycogenolysis is catalyzed in liver, muscle, and brain by tissue-specific isoforms of the enzyme glycogen phosphorylase. This enzyme cleaves the glycogen macromolecule to release glucose-1-phosphate and a new shortened glycogen molecule. Several types of glycogen phosphorylase inhibitors (GPI's) have been reported to date [Martin, J.L. et al. Biochemistry 1991, 30, 10101; Kasvinsky, P.J. et al. J. Biol. Chem. 1978, 253, 3343; Zographos, S. E. et al., Structure, 1997, 11:1413-1425. These compounds, and glycogen phosphorylase inhibitors in general, have been postulated to be of potential use for the treatment of Type 2 diabetes by decreasing hepatic glucose production and lowering glycemia Reported binding sites on the enzyme for inhibitors are the active site, the caffeine or purine binding site and the ATP or nucleotide binding site. The novel binding site described in the instant application is distinct from the active site, the caffeine site or the ATP site.

Human liver glycogen phosphorylase (HLGP) is composed of 2 identical subunits and its activity is regulated by phosphorylation at a single amino acid in each subunit (serine 14). In the phosphorylated form, HLGP is active and, in the unphosphorylated state, the enzyme has very low activity. Thus, attachment of a phosphate group to serine 14 triggers a structural transition in the protein resulting in increased activity. The features of this conformational change have been identified crystallographically in the case of rabbit muscle glycogen phosphorylase (Sprang et al., 1988, Nature 336, 215-221). The experimentally determined HLGP:GPI structure reveals that binding at this site reverses the local structural effects of phosphorylation causing the phosphorylated "active" enzyme to adopt the conformation of the "inactive" unphosphorylated protein. The novel binding site described in this patent is also distinct from the phosphorylation site.

### Brief Description of The Drawings

Figure 1. Tertiary structure of the complete polypeptide of the human liver glycogen phosphorylase a dimer with the part of the enzyme corresponding to the residues listed above identified. The part of the novel binding site

belonging to one subunit is colored purple and that of the two-fold symmetry related subunit is colored blue. Residues of the physiological dimer (other than the novel binding site) from both subunits are colored gold. Figure made using the program Ribbons (Carson, M, (1991), Ribbons 2.0. J. Appl. Cryst. 24 958-961).

Figure 2. Residues of the most preferred novel binding site complexed with a GPI. The novel binding site consists of residues from both subunits. Secondary structural elements from one subunit are colored blue, those from the symmetry related subunit are colored purple. Side chains of residues forming the novel binding site are colored by class: blue, positively charged; green, polar; gold, hydrophobic; red, negatively charged. A molecule of a GPI is shown in ball and stick representation, with gold bonds and atoms colored by type (carbon and chlorine: green; oxygen: red, nitrogen: blue), Figure made using the program Ribbons (Carson, M, (1991), Ribbons 2.0 J. Appl. Cryst. 24 958-961).

Summary of the Invention

In one embodiment, the present invention is directed to a novel binding site for a glycogen phosphorylase inhibitor, in which a portion of said inhibitor is in van der Waals or hydrogen bonding contact with a portion or all portions of the following residues of glycogen phosphorylase:

| parent secondary structure | residue number |
|---|---|
| | 13-23 |
| helix α1 | 24-37 |
| turn | 38-39, 43, 46-47 |
| helix α2 | 48-66, 69-70, 73-74, 76-78 |
| | 79-80 |
| strand β1 | 81-86 |
| | 87-88 |
| strand β2 | 89-92 |
| | 93 |
| helix α3 | 94-102 |
| | 103 |
| helix α4 | 104-115 |
| | 116-117 |
| helix α5 | 118-124 |
| | 125-128 |
| strand β3 | 129-131 |
| | 132-133 |
| helix α6 | 134-150 |
| | 151-152 |
| strand β4 | 153-160 |
| | 161 |
| strand β4b | 162-163 |
| | 164-166 |
| strand β5 | 167-171 |
| | 172-173 |
| strand β6 | 174-178 |
| | 179-190 |
| strand β7 | 191-192 |
| | 194,197 |
| strand β8 | 198-209 |
| | 210-211 |
| strand β9 | 212-216 |
| strand β10 | 219-226, 228-232 |
| | 233-236 |
| strand β11 | 237-239, 241, 243-247 |
| | 248-260 |

(continued)

| parent secondary structure | residue number |
|---|---|
| helix α7 | 261-276 |
| strand β11b | 277-281 |
| reverse turn | 282-289 |
| helix α8 | 290-304 |

A preferred novel binding site for a glycogen phosphorylase inhibitor is that wherein a portion of said inhibitor is in van der Waals or hydrogen bonding contact with a portion or all portions of the following residues of glycogen phosphorylase in one or both subunits:

| parent secondary structure | residue number |
|---|---|
| | 13-23 |
| helix α1 | 24-37 |
| turn | 38-39, 43, 46-47 |
| helix α2 | 48-66, 69-70, 73-74, 76-78 |
| | 79-80 |
| strand β2 | 91-92 |
| | 93 |
| helix α3 | 94-102 |
| | 103 |
| helix α4 | 104-115 |
| | 116-117 |
| helix α5 | 118-124 |
| | 125-128 |
| strand β3 | 129-130 |
| strand β4 | 159-160 |
| | 161 |
| strand β4b | 162-163 |
| | 164-166 |
| strand β5 | 167-168 |
| strand β6 | 178 |
| | 179-190 |
| strand β7 | 191-192 |
| | 194,197 |
| strand β9 | 198-200 |
| strand β10 | 220-226, |
| | 228-232 |
| | 233-236 |
| strand β11 | 237-239, 241, 243-247 |
| | 248-260 |
| helix α7 | 261-276 |
| strand β11b | 277-280 |

A more preferred novel binding site for a glycogen phosphorylase inhibitor is that in which a portion of said inhibitor is in van der Waals or hydrogen bonding contact with a portion or all portions of the following residues of glycogen phosphorylase in one or both subunits:

| residue number |
|---|
| 33-39 |
| 49-66 |
| 94 |

(continued)

| residue number |
|---|
| 98 |
| 102 |
| 125-126 |
| 160 |
| 162 |
| 182-192 |
| 197 |
| 224-226 |
| 228-231 |
| 238-239 |
| 241 |
| 245 |
| 247 |

A most preferred novel binding site for a glycogen phosphorylase inhibitor is that wherein a portion of said inhibitor is in van der Waals or hydrogen bonding contact with a portion or all portions of the following residues of glycogen phosphorylase in one or both subunits:

| residue number |
|---|
| 37-39 |
| 53 |
| 57 |
| 60 |
| 63-64 |
| 184-192 |
| 226 |
| 229 |

In another embodiment, the present invention is directed to a complex of glycogen phosphorylase with a glycogen phosphorylase inhibitor which binds at the binding site.
In still another embodiment, the present invention is directed to a crystal of a complex of glycogen phosphorylase with a glycogen phosphorylase inhibitor which binds at the binding site.
In yet still another embodiment, the present invention is directed to a crystal of a complex of glycogen phosphorylase with a glycogen phosphorylase inhibitor wherein said glycogen phosphorylase inhibitor capable of binding to this site is of Formulae I, II and III as set forth in the Detailed Description of the Invention.
In another embodiment, the present invention is directed to a crystal of a complex of glycogen phosphorylase with a glycogen phosphorylase inhibitor which binds at the binding site wherein the glycogen phosphorylase is human liver glycogen phosphorylase and the crystal has

the following physical measurements:

| | |
|---|---|
| Space group | $P3_1$ |
| Unit Cell: | a = b = 124.63 angstroms<br>c = 124.06 angstroms<br>$\alpha = \beta$ = 90.00 degrees<br>$\delta$ = 120.00 degrees |

said values measured in a unit cell having the following variability of the dimensions.

| | | |
|---|---|---|
| a | 123.4 - | 124 63 Å (1%) |
| c | 122.22 - | 124.06 Å (1.5%) |

In another embodiment, the present invention is directed to a method of detecting the formation of a complex

of glycogen phosphorylase with a glycogen phosphorylase inhibitor which binds at the binding site.

In still another embodiment, the present invention is directed to a method for detecting the formation of a complex of glycogen phosphorylase with a glycogen phosphorylase inhibitor which binds at the binding site which relies on displacement of a bound ligand from the binding site.

In yet still another embodiment, the present invention is directed to a method of determining the inhibition of human liver glycogen phosphorylase which is based on formation of a complex of glycogen phosphorylase with a glycogen phosphorylase inhibitor which binds at the binding site.

Another embodiment of the present invention is a method for designing an inhibitor of human liver glycogen phosphorylase, said method comprising steps a-c below:

a) forming a complex of a compound binding to glycogen phosphorylase at the novel binding site
b) determining structural features of the complex in step (a), in particular those of the binding site for said compound, and
c) using the structural features determined in step (b), in particular those of the binding site for said compound of step (a), or a model based on said structural features, to design a human liver glycogen phosphorylase inhibitor capable of binding to the novel binding site.

Another embodiment of the present invention is a method of treatment of a mammal having hyperglycemia, hyperinsulimemia, hyperlipidemia, insulin resistance or tissue ischemia comprising:

a) forming a complex of a compound binding to glycogen phosphorylase at the novel binding site
b) determining structural features of the complex in step (a), in particular those of the binding site for said compound and
c) using the structural features determined in step (b), in particular those of the binding site for said compound of step (a), or a model based on said structural features, to design a human liver glycogen phosphorylase inhibitor capable of binding to the novel binding site.
d) administering the compound of step c) to a patient in need of such treatment.

## Detailed Description Of The Invention

The present invention is directed to a novel binding site on glycogen phosphorylase for glycogen phosphorylase inhibitors. A glycogen phosphorylase inhibitor is any substance which, when present together with glycogen phosphorylase, retards or reduces the activity of glycogen phosphorylase. This novel binding site was revealed by the determination of the 3-dimensional structure of glycogen phosphorylase (GP) complexed with a series of classes of glycogen phosphorylase inhibitors. The novel binding site is unexpected and previously unknown. The structure of the inhibited enzyme with an inhibitor bound at the novel binding site may explain how GPI's binding at this site inhibit the enzyme and also allows the design of other GPIs which may bind to this site. Without this structure, the structure of a similar complex, or other knowledge of this site, the novel binding site would not be chosen as either a target of or a basis for designing glycogen phosphorylase inhibitors by those skilled in the art. The secondary structural elements that form this novel binding site are conserved in the known crystal structures of phosphorylases from yeast, rabbit muscle and human liver. The novel binding site is present in all known mammalian species of phosphorylase.

Crystallization of the complex and determination of the structure of the complex allows for the determination of the amino acids and the location of the amino acids which determine the binding site for the glycogen phosphorylase inhibitor. It will be understood that favorable hydrogen bonding interactions between glycogen phosphorylase and a glycogen phosphorylase inhibitor may occur directly or through a water molecule.

The steps for determination of the complex are as follows. It will be understood that this is merely illustrative of the instant invention and that other glycogen phosphoryalse inhibitors binding at the novel binding site and or glucose or other glucose analogs, may be employed.

## Expression and fermentation

The HLGP, and HMGP cDNAs are expressed from plasmid pKK233-2 (Pharmacia Biotech. Inc., Piscataway, New Jersey) in *E. coli* strain XL-1 Blue (Stratagene Cloning Systems, La Jolla, CA). The strain is inoculated into LB medium (consisting of 10 g tryptone, 5 g yeast extract, 5 g NaCl, and 1 ml 1N NaOH per liter) plus 100 mg/L ampicillin, 100 mg/L pyridoxine and 600 mg/L $MnCl_2$ and grown at 37°C to a cell density of $OD_{550}$ = 1.0. At this point, the cells are induced with 1 mM isopropyl-1-thio-β-D-galactoside (IPTG). Three hours after induction the cells are harvested by centrifugation and cell pellets are frozen at -70°C until needed for purification.

The HBGP cDNA can be expressed by several methodologies, for example, by the method described by

Crerar, et al. (J. Biol. Chem. 270:13748-13756) as follows: the HBGP cDNA can be expressed from plasmid pTACTAC in *E. Coli* strain 25A6 of plasmid. The strain is inoculated into LB medium (consisting of 10 g tryptone, 5 g yeast extract, 5 g NaCl, and 1 ml 1 N NaOH per liter) plus 50 mg/L ampicillin and grown overnight, then resuspended in fresh LB medium plus 50 mg/L ampicillin, and inoculated into a 40X volume of LB/amp media containing 250 µM isopropyl-1-thio-β-D-galactoside (IPTG), 0.5 mM pyridoxine and 3 mM $MnCl_2$ and grown at 22°C for 48-50 hours. The cells can then be harvested by centrifugation and cell pellets are frozen at -70°C until needed for purification.

The HLGP cDNA is also expressed from plasmid pBlueBac III (Invitrogen Corp., San Diego, CA) which is cotransfected with BaculoGold Linear Viral DNA (Pharmingen, San Diego, CA) into Sf9 cells. After several rounds of plaque purification, a virus containing the HLGP encoding DNA was isolated and amplified. Expression was confirmed by western blot and enzyme activity assays. Optimized expression of the HLGP was carried out in Sf900 II media (GIBco-BRL) under serum free conditions in Sf9 cells at a multiplicity of infections of O.5 for 22 hours and at a cell density of $2x10^6$ cells/ml. After growth for 72 hours at 27°C, cells are centrifuged, and the cell pellets frozen at -70°C until needed for purification.

Purification of Glycogen Phosphorylase expressed in *E. coli* or SF9 cells

The *E. coli* cells in pellets described above are resuspended in 25 mM β-glycerophosphate (pH 7.0) with 0.2 mM DTT, 1 mM $MgCl_2$, plus the following protease inhibitors:

| | |
|---|---|
| 0.7 µg/mL | Pepstatin A |
| 0.5 µg/mL | Leupeptin |
| 0.2 mM | phenylmethylsulfonyl fluoride (PMSF), and |
| 0.5 mM | EDTA, |

lysed by pretreatment with 200 µg/mL lysozyme and 3 µg/mL DNAase followed by sonication in 250 mL batches for 5 x 1.5 minutes on ice using a Branson Model 450 ultrasonic cell disrupter (Branson Sonic Power Co., Danbury CT). The *E. coli* cell lysates are then cleared by centrifugation at 35,000 X g for one hour followed by filtration through 0.45 micron filters. GP in the soluble fraction of the lysates (estimated to be less than 1% of the total protein) is purified by monitoring the enzyme activity (as described in GPa Activity Assay section, below) during the following steps (detailed below) in the following order: metal affinity chromatography, 5' AMP Sepharose chromatography, dye-ligand chromatography and anion exchange chromatography.

Sf9 cells in pellets described above are resuspended to a buffer volume: cell mass ratio of 3:1, in buffer consisting of 25 mM β-glycerophosphate (pH 7.0) with 0.2 mM DTT, 1 mM MgCl2, plus the following protease inhibitors:

| | |
|---|---|
| 0.7 µg/mL | Pepstatin A |
| 0.5 µg/mL | Leupeptin |
| 0.2 mM | phenylmethylsulfonyl fluoride (PMSF), and |
| 0.5 mM | EDTA, |

lysed by pretreatment with 3 µg/mL DNAase followed by sonication in batches for 3 x 1 minutes on ice using a Branson Model 450 ultrasonic cell disrupter (Branson Sonic Power Co., Danbury, CT). The Sf9 cell lysates are then cleared by centrifugation at 35,000 X g for one hour followed by filtration through 0.45 micron filters. GP in the soluble fraction of the lysates (estimated to be 1.5% of the total protein) is purified by monitoring the enzyme activity (as described in GPa Activity Assay section) during metal affinity chromatography followed by anion exchange chromatography.

Metal Affinity Chromatography

This step is based on the method of Luong et al (Luong et al. Journal of Chromatography (1992) 584, 77-84.). 500 mL of the filtered soluble fraction of cell lysates (prepared from approximately 160 - 250 g of original cell pellet) are loaded onto a 130 mL column of IMAC Chelating-Sepharose (Pharmacia LKB Biotechnology, Piscataway, New Jersey) which has been charged with 50 mM $CuCl_2$ and 25 mM β-glycerophosphate, 250 mM NaCl and 1 mM imidazole at pH 7 (equilibration buffer). The column is washed with equilibration buffer until the $A_{280}$ returns to baseline. The sample is then eluted from the column with a gradient of 0-400 mM imidazole. Fractions containing the GP activity are pooled (approximately 600 mL), and ethylenediaminetetraacetic acid (EDTA), DL-dithiothreitol (DTT), phenylmethylsulfonyl fluoride (PMSF), leupeptin and pepstatin A are added to obtain 0.3 mM, 0.2 mM, 0.2 mM, 0.5 µg/mL and 0.7

µg/mL concentrations respectively. The pooled GP is desalted over a Sephadex G-25 column (Sigma Chemical Co., St. Louis, Missouri) equilibrated with 25 mM Tris-HCl (pH 7.3), 3 mM DTT buffer (Buffer A) to remove imidazole and is stored on ice.

5'- AMP-Sepharose Chromatography

The desalted pooled GP sample from the metal affinity step (approximately 600mL) is next mixed with 70 mL of 5'-AMP Sepharose (Pharmacia LKB Biotechnology, Piscataway, New Jersey) which has been equilibrated with Buffer A (see above). The mixture is gently agitated for one hour at 22°C then packed into a column and washed with Buffer A until the $A_{280}$ returns to baseline. GP and other proteins are eluted from the column with 25 mM Tris-HCl, 0.2 mM DTT and 10 mM adenosine 5'-monophosphate (AMP) at pH 7.3 (Buffer B). GP-containing fractions are pooled following identification by determining enzyme activity (described below) and visualizing the $M_r$ approximately 97 kd GP protein band by sodium dodecyl sulfate polyacrylamide gel electrophoresis (SDS-PAGE) followed by silver staining (2D-silver Stain II "Daiichi Kit", Daiichi Pure Chemicals Co., LTD., Tokyo, Japan) and then pooled. The pooled GP is dialyzed into 25 mM β-glycerophosphate, 0.2 mM DTT, 0.3 mM EDTA, 200 mM NaCl, pH 7.0 buffer (Buffer C) and stored on ice until use.

Dye-Ligand Affinity Chromatography

The HLGP containing fractions from 5-AMP-Sepharose Chromatography are pooled and dialyzed against 25mM β-glycerophosphate, 0.2M NaCl, 0.5mM DTT, 0.5mM EDTA, pH 7.0 (DL-Buffer). The pool is loaded onto a Matrex Green A agarose column (Amicon) equilibrated with DL-Buffer. The bound proteins are eluted with 20mM 5'-AMP in DL-buffer and the HLGP-containing fractions are pooled, and diaconcentrated in an Amicon stirred cell unit against Buffer A to simultaneoulsy concentrate and remove AMP.

Conversion of HLGPb to HLGPa

Partially purified preparations of HLGP from either *E. coli* or Sf9 cell expression are converted fully to the phosphorylated a form (described below) prior to anion exchange chromatography .

Anion Exchange Chromatography

Following activation of GPb to GPa by reaction with the immobilized phosphorylase kinase, as described below, the pooled GPa fractions are dialyzed against 25 mM Tris-HCl, pH 7.5, containing 0.5 mM DTT, 0.2 mM EDTA, 1.0 mM phenylmethylsulfonyl fluoride (PMSF), 1.0 µg/mL leupeptin and 1.0 µg/mL pepstatin A. The fraction is then loaded onto a Resource Q or a MonoQ Anion Exchange Chromatography column (Pharmacia Biotech. Inc., Piscataway, New Jersey). The column is washed with equilibration buffer until the $A_{280}$ returns to baseline. The sample is then eluted from the column with a linear gradient of 0-0.25 M NaCl to remove the bound GP and other bound proteins. GP-containing fractions elute between 0.1-0.2 M NaCl range, as detected by monitoring the eluant for peak protein absorbance at $A_{280}$. The GP protein is then identified by visualizing the $M_r$ approximately 97 kd GP protein band by sodium dodecyl sulfate polyacrylamide gel electrophoresis (SDS-PAGE) followed by silver staining (2D-silver Stain II "Daiichi Kit", Daiichi Pure Chemicals Co., LTD., Tokyo, Japan) and then pooled. The pooled GP is dialyzed into 25 mM N,N-bis-(2-hydroxyethyl)-2-aminoethanesulfonic acid (BES), 1.0 mM DTT, 0.5 mM EDTA, 5 mM NaCl, pH 6.8 buffer and stored on ice until use.

Determination of GP Enzyme Activity

Activation of GP: Conversion of GPb to GPa

Prior to the determination of GP enzyme activity, the enzyme is converted from the inactive form as expressed in *E. coli* strain XL-1 Blue (designated GPb) (Stragene Cloning Systems, La Jolla, California), to the active form (designated GPa) by phosphorylation of GP using phosphorylase kinase as follows. The fraction of inactive enzyme as expressed in Sf9 cells (designated GPb) is also converted to the active form (designated GPa) by the following procedure.

GP reaction with Immobilized Phosphorylase Kinase

Phosphorylase kinase (Sigma Chemical Company, St. Louis, MO) is immobilized on Affi-Gel® 10 (BioRad

Corp., Melvile, NY) as per the manufacturer's instructions. In brief, the phosphorylase kinase enzyme (10 mg) is incubated with washed Affi-Gel® beads (1 mL) in 2.5 mL of 100 mM HEPES and 80 mM $CaCl_2$ at pH 7.4 for 4 hours at 4°C. The Affi-Gel® beads are then washed once with the same buffer prior to blocking with 50 mM HEPES and 1 M glycine methyl ester at pH 8.0 for one hour at room temperature. Blocking buffer is removed and replaced with 50 mM HEPES (pH 7.4), 1 mM β-mercaptoethanol and 0.2% $NaN_3$ for storage. Prior to use, the Affi-Gel® immobilized phosphorylase kinase beads are equilibrated by washing in the buffer used to perform the kinase reaction, consisting of 25 mM β-glycerophosphate, 0.3 mM DTT, and 0.3mM EDTA at pH 7.8 (kinase assay buffer).

The inactive GPb obtained from the chromatograhic procedure above is diluted 1:10 with the kinase assay buffer then mixed with the aforementioned phosphorylase kinase enzyme immobilized on the Affi-Gel® beads. NaATP is added to 7 mM and $MgCl_2$ to 22 mM. The resulting mixture is mixed gently at 25°C for 30 to 60 minutes. The sample is removed from the beads and the percent activation of GPb by conversion to GPa is estimated by determining GP enzyme activity in the presence and absence of 3.3 mM AMP. The percentage of total GP enzyme activity due to GPa enzyme activity (AMP-independent) is then calculated as follows:

$$\text{\% of total HLGPa} = \frac{\text{HLGP activity - AMP}}{\text{HLGP activity + AMP}} \times 100$$

Alternately, the conversion of GPb to GPa can be monitored by isoelectric focusing, based on the shift in electrophoretic mobility that is noted following conversion of GPb to GPa. GP samples are analyzed by isoelectric focusing (IEF) utilizing the Pharmacia PfastGel System (Pharmacia Biotech. Inc., Piscataway, New Jersey) using precast gels (pl range 4-6.5) and the manufacturer's recommended method. The resolved GPa and GPb bands are then visualized on the gels by silver staining (2D-silver Stain II "Daiichi Kit", Daiichi Pure Chemicals Co., LTD., Tokyo, Japan). Identification of GPa and GPb is made by comparison to *E. coli* derived GPa and GPb standards that are run in parallel on the same gels as the experimental samples. The activated enzyme pool was dialyzed prior to ion exchange.

GPa Activity Assay

The disease/condition treating/preventing activities described herein of the compounds of this invention can be indirectly determined by assessing the effect of the compounds of this invention on the activity of the activated form of glycogen phosphorylase (GPa) by one of two methods; glycogen phosphorylase a activity is measured in the forward direction by monitoring the production of glucose-1-phosphate from glycogen or by following the reverse reaction, measuring glycogen synthesis from glucose-1-phosphate by the release of inorganic phosphate. All reactions can run in triplicate in 96-well microtiter plates and the change in absorbance due to formation of the reaction product is measured at the wavelength specified below in a MCC/340 MKII Elisa Reader (Lab Systems, Finland), connected to a Titertech Microplate Stacker (ICN Biomedical Co, Huntsville, Alabama).

To measure the GPa enzyme activity in the forward direction, the production of glucose-1-phosphate from glycogen is monitored by the multienzyme coupled general method of Pesce et al. [Pesce, M.A., Bodourian, S.H., Harris, R.C. and Nicholson, J.F. (1977) Clinical Chemistry 23, 1711-1717] modified as follows: 1 to 100 μg GPa, 10 units phosphoglucomutase and 15 units glucose-6-phosphate dehydrogenase (Boehringer Mannheim Biochemicals, Indianapolis, IN) are diluted to 1 mL in Buffer D (pH 7.2, 50 mM HEPES, 100 mM KCl, 2.5 mM ethyleneglycoltetraacetic acid (EGTA), 2.5 mM $MgCl_2$, 3.5 mM $KH_2PO_4$ and 0.5 mM dithiothreitol). 20 μl of this stock is added to 80 μl of Buffer D containing 0.47 mg/mL glycogen, 9.4 mM glucose, 0.63 mM of the oxidized form of nicotinamide adenine dinucleotide phosphate (NADP+). The compounds to be tested are added as 5 μL of solution in 14% dimethylsulfoxide (DMSO) prior to the addition of the enzymes. The basal rate of GPa enzyme activity in the absence of inhibitors is determined by adding 5 μL of 14% DMSO and a fully-inhibited rate of GPa enzyme activity is obtained by adding 20 μL of 50 mM of the positive control test substance, caffeine. The reaction is followed at room temperature by measuring the conversion of oxidized NADP+ to reduced NADPH at 340 nm.

To measure the GPa enzyme activity in the reverse direction, the conversion of glucose-1-phosphate into glycogen plus inorganic phosphate is measured by the general method described by Engers et al. [Engers, H.D., Shechosky, S. and Madsen, N.B. (1970) Can. J. Biochem. 48, 746-754] modified as follows: 1 to 100 μg GPa is diluted to 1 mL in Buffer E (pH 7.2, 50 mM HEPES, 100 mM KCl, 2.5 mM EGTA, 2.5 mM $MgCl_2$ and 0.5 mM dithiothreitol). 20 μL of this stock is added to 80 μL of Buffer E with 1.25 mg/mL glycogen, 9.4 mM glucose, and 0.63 mM glucose-1-phosphate. The compounds to be tested are added as 5 μL of solution in 14% DMSO prior to the addition of the enzyme. The basal rate of GPa enzyme activity in the absence of added inhibitors is determined by adding 5 μL of 14% DMSO. The fully-inhibited rate of GPa enzyme activity is obtained by adding 20 μL of 50 mM caffeine. This mixture is incubated at room temperature for 1 hour and the inorganic phosphate released from the glucose-1-phosphate is measured by the general method of Lanzetta et al. [Lanzetta, P.A., Alvarez, L.J., Reinach, P.S. and Candia, O.A. (1979) Anal. Biochem. 100, 95-97] modified as follows: 150 μL of 10 mg/mL ammonium molybdate, 0.38 mg/mL malachite

green in 1 N HCl is added to 100 µL of the enzyme mix. After a 20 minute incubation at room temperature, the absorbance is measured at 620 nm.

The above assays carried out with a range of concentrations of test compound allows the determination of an $IC_{50}$ value (concentration of test compound required for 50% inhibition) for the *in vitro* inhibition of GPa enzyme activity by that test compound.

Example 1.

**Crystallization**

Recombinant human liver glycogen phosporylase protein, as prepared above, was concentrated to 30 mg/mL in 20 mM Na BES + 1.0 mM EDTA + 0.5 mM DTT, pH 6.8. Reservoir solution = 0.1 M NaMES + 25% MPD (v/v). The glycogen phosphorylase inhibitor, (S)-1-{2-[(5-chloro-1H-indole-2-carbonyl)-amino]-3-phenyl-propionyl}-azetidine-3-carboxylic acid was added to the protein : N-acetyl-βD-glucopyranosylamine (GlcNAc) complex at a molar ratio of 6:1. Hanging drops (vapour diffusion method) were set up using 2 µl complex + 2 µl reservoir solution. Crystals were grown at 17°C. N-acetyl-B-D-glucopyranosylamine was prepared and purchased from Dr. George Fleet (Oxford University, United Kingdom). Depending on the compound used, GlcNAc may be omitted without altering the quality of the crystals.

**Crystal**

Space group: $P3_1$. Unit cell: a = b = 124.63 angstroms, c = 124.06 angstroms, $\alpha = \beta = 90.0$ degrees, $\gamma = 120.0$ degrees. The asymmetric unit of the crystal contains the functional dimer of human liver phosphorylase. Each subunit binds 1 molecule of the above mentioned glycogen phosphorylase inhibitor and additionally binds one molecule of 1-GlcNAc in the crystal. It will be understood that if another glycogen phosphorylase inhibitor which binds at the novel binding site or other glucose analog is employed, that said glycogen phosphorylase inhibitor and/or said glucose analog will be present in the crystal.

**Data Measurement**

Crystals were transferred using a nylon loop from the hanging drop to a crystallization solution (consisting of the equilibrated reservoir solution to which 25 mM GlcNAc and 1mM (S)-1-{2-[(5-chloro-1H-indole-2-carbonyl)-amino]-3-phenyl-propionyl}-azetidine-3-carboxylic acid sodium salt was added briefly and then placed directly into a stream of liquid nitrogen gas at 108K. for data measurement. The data were measured using a MaRResearch Detector equipped with Supper focusing mirrors mounted on a rotating anode operating at 100mA and 50 Kv. 200 frames of 0.75 degree oscillations were measured. The data were processed using XDISPLAYF, Denzo and Scalepack, (Z. Otwinowski and W. Minor, Processing of X-ray Diffraction Data Collected in Oscillation Mode, Methods in Enzymology, Volume 276:Macromolecular Crystallography, Part. A, p. 307-326, 1997, C.W. Carter, Jr. and R. M. Sweet, Eds., Academic Press.)

**Solution of the Structure**

The structure was solved by the method of molecular replacement as implemented in X-PLOR [PUT IN REFERENCE] using the known coordinates of human liver phosphorylase *a* complexed with AMP. AMP was omitted and a polyalanine chain was used as a search molecule. The results of a rotation solution calculated using data from 10 - 4 Å resolution were further optimized using Patterson Correlation refinement reference. The molecule was divided up into four rigid bodies; one for each of the N- and C-terminal domains of each subunit. The second highest solution from Patterson Correlation refinement resulted in a solution with a free R factor of 47%. A translation function calculated with this solution in both space groups $P3_1$ and $P3_2$ resulted in a an interpretable map. After Powell minimization, the free R factor dropped to 41.3% in space group $P3_1$. The model was rebuilt using the graphics program ○ and refined in X-PLOR, using the maximum likelihood target.

Certain compounds able to bind to this site in vivo have the structure:

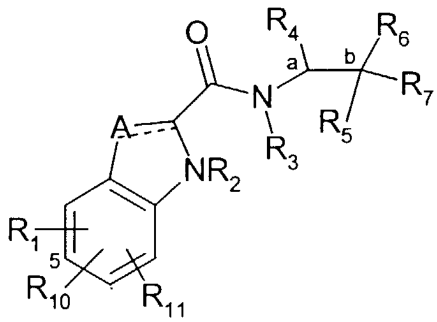

Formula I

and the pharmaceutically acceptable salts and prodrugs thereof
wherein
the dotted line (---) is an optional bond;

A is -C(H)=, -C(($C_1$-$C_4$)alkyl)= or -C(halo)= when the dotted line (---) is a bond, or A is methylene or -CH(($C_1$-$C_4$) alkyl)- when the dotted line (---) is not a bond;
$R_1$, $R_{10}$ or $R_{11}$ are each independently H, halo, 4-, 6- or 7-nitro, cyano, ($C_1$-$C_4$)alkyl, ($C_1$-$C_4$)alkoxy, fluoromethyl, difluoromethyl or trifluoromethyl;
$R_2$ is H;
$R_3$ is H or ($C_1$-$C_5$)alkyl;
$R_4$ is methyl, ethyl, n-propyl, hydroxy($C_1$-$C_3$)alkyl, ($C_1$-$C_3$)alkoxy($C_1$-$C_3$)alkyl, phenyl($C_1$-$C_4$)alkyl, phenylhydroxy ($C_1$-$C_4$)alkyl, phenyl($C_1$-$C_4$)alkoxy($C_1$-$C_4$)alkyl, thien-2- or -3-yl($C_1$-$C_4$)alkyl or fur-2- or -3-yl($C_1$-$C_4$)alkyl wherein said $R_4$ rings are mono-, di- or tri-substituted independently on carbon with H, halo, ($C_1$-$C_4$)alkyl, ($C_1$-$C_4$)alkoxy, trifluoromethyl, hydroxy, amino or cyano; or
$R_4$ is pyrid-2-, -3- or -4-yl($C_1$-$C_4$)alkyl, thiazol-2-, -4- or -5-yl($C_1$-$C_4$)alkyl, imidazol -1-,-2-, -4- or -5-yl($C_1$-$C_4$)alkyl, pyrrol-2- or -3-yl($C_1$-$C_4$)alkyl, oxazol-2-, -4- or -5-yl-($C_1$-$C_4$)alkyl, pyrazol-3-, -4- or -5-yl($C_1$-$C_4$)alkyl, isoxazol-3-, -4- or -5-yl($C_1$-$C_4$)alkyl, isothiazol-3-, -4- or -5-yl($C_1$-$C_4$)alkyl, pyridazin-3- or -4-yl-($C_1$-$C_4$)alkyl, pyrimidin-2-, -4-, -5- or -6-yl($C_1$-$C_4$)alkyl, pyrazin-2- or -3-yl($C_1$-$C_4$)alkyl or 1,3,5-triazin-2-yl($C_1$-C4)alkyl, wherein said preceding $R_4$ heterocycles are optionally mono- or di-substituted independently with halo, trifluoromethyl, ($C_1$-$C_4$)alkyl, ($C_1$-$C_4$) alkoxy, amino or hydroxy and said mono-or di-substituents are bonded to carbon;
$R_5$ H, hydroxy, fluoro, ($C_1$-$C_5$)alkyl, ($C_1$-$C_5$)alkoxy, ($C_1$-$C_6$)alkanoyl, amino($C_1$-$C_4$)alkoxy, mono-N- or di-N,N-($C_1$-$C_4$)alkylamino($C_1$-$C_4$)alkoxy, carboxy($C_1$-$C_4$)alkoxy, ($C_1$-$C_5$)alkoxy-carbonyl($C_1$-$C_4$)alkoxy, benzyloxycarbonyl ($C_1$-$C_4$)alkoxy, or carbonyloxy wherein said carbonyloxy is carbon-carbon linked with phenyl, thiazolyl, imidazolyl, 1H-indolyl, furyl, pyrrolyl, oxazolyl, pyrazolyl, isoxazolyl, isothiazolyl, pyridazinyl, pyrimidinyl, pyrazinyl or 1,3,5-triazinyl and wherein said preceding $R_5$ rings are optionally mono-substituted with halo, ($C_1$-$C_4$)alkyl, ($C_1$-$C_4$)alkoxy, hydroxy, amino or trifluoromethyl and said mono-substituents are bonded to carbon;
$R_7$ is H, fluoro or ($C_1$-$C_5$)alkyl; or
$R_5$ and $R_7$ can be taken together to be oxo;
$R_4$ is carboxy, or ($C_1$-$C_8$)alkoxycarbonyl,

wherein

$R_8$ is ($C_1$-$C_3$)alkyl, hydroxy or ($C_1$-$C_3$)alkoxy; and
$R_9$ is H, ($C_1$-$C_8$)alkyl, hydroxy, ($C_1$-$C_8$)alkoxy, methylene-perfluorinated($C_1$-$C_8$)alkyl, phenyl, pyridyl, thienyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, pyridazinyl, pyrimidinyl, pyrazinyl, piperazinyl or 1,3,5-triazinyl wherein said preceding $R_9$ rings are carbon-nitrogen linked; or
$R_9$ is mono-, di- or tri-substituted ($C_1$-$C_5$)alkyl, wherein said substituents are independently H, hydroxy, amino, mono-N- or di-N,N-($C_1$-$C_5$)alkylamino; or
$R_9$ is mono- or di-substituted ($C_1$-$C_5$)alkyl, wherein said substituents are independently phenyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl or 1,3,5-triazinyl
wherein the nonaromatic nitrogen-containing $R_9$ rings are optionally mono-substituted on nitrogen with ($C_1$-$C_6$)

alkyl, benzyl, benzoyl or $(C_1-C_6)$alkoxycarbonyl and wherein the $R_9$ rings are optionally mono-substituted on carbon with halo, $(C_1-C_4)$alkyl, $(C_1-C_4)$alkoxy, hydroxy, amino, or mono-N- and di-N,N $(C_1-C_5)$alkylamino provided that no quaternized nitrogen is included and there are no nitrogen-oxygen, nitrogen-nitrogen or nitrogen-halo bonds;

$R_{12}$ is piperazin-1-yl, 4-$(C_1-C_4)$alkylpiperazin-1-yl, 4-formylpiperazin-1-yl, morpholino, thiomorpholino, 1-oxothiomorpholino, 1,1-dioxo-thiomorpholino, thiazolidin-3-yl, 1-oxothiazolidin-3-yl, 1,1-dioxo-thiazolidin-3-yl, 2-$(C_1-C_6)$alkoxycarbonylpyrrolidin-1-yl, oxazolidin-3-yl or 2(R)-hydroxymethylpyrrolidin-1-yl; or

$R_{12}$ is 3- and/or 4-mono-or di-substituted oxazetidin-2-yl, 2-, 4-, and/or 5- mono- or di-substituted oxazolidin-3-yl, 2-, 4-, and/or 5- mono- or di- substituted thiazolidin-3-yl, 2-, 4-, and/or 5- mono- or di- substituted 1-oxothiazolidin-3-yl, 2-, 4-, and/or 5- mono- or di-substituted 1,1-dioxothiazolidin-3-yl, 3- and/or 4-, mono- or di-substituted pyrrolidin-1-yl, 3-, 4-and/or 5-, mono-, di- or tri-substituted piperidin-1-yl, 3-, 4-, and/or 5- mono-, di-, or tri-substituted piperazin-1-yl, 3-substituted azetidin-1-yl, 4- and/or 5-, mono- or di-substituted 1,2-oxazinan-2-yl, 3-and/or 4-mono- or di-substituted pyrazolidin-1-yl, 4- and/or 5-, mono- or di-substituted isoxazolidin-2-yl, 4- and/or 5-, mono- and/or di-substituted isothiazolidin-2-yl wherein said $R_{12}$ substituents are independently H, halo, $(C_1-C_5)$-alkyl, hydroxy, amino, mono-N- or di-N,N-$(C_1-C_5)$alkylamino, formyl, oxo, hydroxyimino, $(C_1-C_5)$alkoxy, carboxy, carbamoyl, mono-N-or di-N,N-$(C_1-C_4)$alkylcarbamoyl, $(C_1-C_4)$alkoxyimino, $(C_1-C_4)$alkoxymethoxy, $(C_1-C_6)$alkoxycarbonyl, carboxy$(C_1-C_5)$alkyl or hydroxy$(C_1-C_5)$alkyl;

with the proviso that if $R_4$ is H, methyl, ethyl or n-propyl, $R_5$ is OH;

with the proviso that if $R_5$ and $R_7$ are H, then $R_4$ is not H, methyl, ethyl, n-propyl, hydroxy$(C_1-C_3)$alkyl or $(C_1-C_3)$alkoxy$(C_1-C_3)$alkyl and $R_6$ is $C(O)NR_8R_9$, $C(O)R_{12}$ or $(C_1-C_4)$alkoxycarbonyl.

Compounds of formula I are disclosed in published Patent Cooperation Treaty Application number WO 96/39385, the complete disclosure of which is hereby incorporated by reference.

Other compounds able to bind to this site in vivo have the structure:

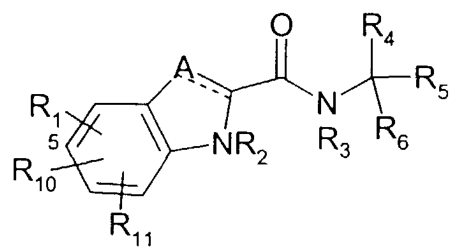

Formula II

and the pharmaceutically acceptable salts and prodrugs thereof
wherein
the dotted line (---) is an optional bond;

A is -C(H)=, -C($(C_1-C_4)$alkyl)=, -C(halo)= or -N=, when the dotted line (---) is a bond, or A is methylene or -CH($(C_1-C_4)$alkyl)-, when the dotted line (---) is not a bond;

$R_1$, $R_{10}$ or $R_{11}$ are each independently H, halo, cyano, 4-, 6-, or 7-nitro, $(C_1-C_4)$alkyl, $(C_1-C_4)$alkoxy, fluoromethyl, difluoromethyl or trifluoromethyl;

$R_2$ is H;

$R_3$ is H or $(C_1-C_5)$alkyl;

$R_4$ is H, methyl, ethyl, n-propyl, hydroxy$(C_1-C_3)$alkyl, $(C_1-C_3)$alkoxy$(C_1-C_3)$alkyl, phenyl$(C_1-C_4)$alkyl, phenylhydroxy$(C_1-C_4)$alkyl, (phenyl)($(C_1-C_4)$-alkoxy)$(C_1-C_4)$alkyl, thien-2-or -3-yl$(C_1-C_4)$alkyl or fur-2- or -3-yl$(C_1-C_4)$alkyl wherein said $R_4$ rings are mono-, di- or tri-substituted independently on carbon with H, halo, $(C_1-C_4)$alkyl, $(C_1-C_4)$alkoxy, trifluoromethyl, hydroxy, amino, cyano or 4,5-dihydro-1 H-imidazol-2-yl; or

$R_4$ is pyrid-2-, -3- or -4-yl$(C_1-C_4)$alkyl, thiazol-2-, -4- or -5-yl$(C_1-C_4)$alkyl, imidazol-2-,-4- or -5-yl$(C_1-C_4)$alkyl, pyrrol-2- or -3-yl$(C_1-C_4)$alkyl, oxazol-2-, -4- or -5-yl$(C_1-C_4)$alkyl, pyrazol-3-, -4- or -5-yl$(C_1-C_4)$alkyl, isoxazol-3-, -4- or -5-yl$(C_1-C_4)$alkyl, isothiazol-3-, -4- or -5-yl$(C_1-C_4)$alkyl, pyridazin-3- or -4-yl$(C_1-C_4)$alkyl, pyrimidin-2-, -4-, -5- or -6-yl$(C_1-C_4)$alkyl, pyrazin-2- or -3-yl$(C_1-C_4)$alkyl, 1,3,5-triazin-2-yl$(C_1-C_4)$alkyl or indol-2-$(C_1-C_4)$alkyl, wherein said preceding $R_4$ heterocycles are optionally mono- or di-substituted independently with halo, trifluoromethyl, $(C_1-C_4)$alkyl, $(C_1-C_4)$alkoxy, amino, hydroxy or cyano and said substituents are bonded to carbon; or

$R_4$ is $R_{15}$-carbonyloxymethyl, wherein said $R_{15}$ is phenyl, thiazolyl, imidazolyl, 1H-indolyl, furyl, pyrrolyl, oxazolyl, pyrazolyl, isoxazolyl, isothiazolyl, pyridyl, pyridazinyl, pyrimidinyl, pyrazinyl or 1,3,5-triazinyl and wherein said preceding $R_{15}$ rings are optionally mono- or di-substituted independently with halo, amino, hydroxy, $(C_1-C_4)$alkyl, $(C_1-C_4)$alkoxy or trifluoromethyl and said mono- or di-substituents are bonded to carbon;

$R_5$ is H, methyl, ethyl, n-propyl, hydroxymethyl or hydroxyethyl;

$R_6$ is carboxy, $(C_1-C_8)$alkoxycarbonyl, benzyloxycarbonyl, $C(O)NR_8R_9$ or $C(O)R_{12}$

wherein $R_8$ is H, $(C_1-C_6)$alkyl, cyclo$(C_3-C_6)$alkyl, cyclo$(C_3-C_6)$alkyl$(C_1-C_5)$alkyl, hydroxy or $(C_1-C_8)$alkoxy; and

$R_9$ is H, cyclo$(C_3-C_8)$alkyl, cyclo$(C_3-C_8)$alkyl$(C_1-C_5)$alkyl, cyclo$(C_4-C_7)$alkenyl, cyclo$(C_3-C_7)$alkyl$(C_1-C_5)$alkoxy, cyclo$(C_3-C_7)$alkyloxy, hydroxy, methylene-perfluorinated$(C_1-C_8)$alkyl, phenyl, or a heterocycle wherein said heterocycle is pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl, 1,3,5-triazinyl, benzothiazolyl, benzoxazolyl, benzimidazolyl, thiochromanyl or tetrahydrobenzothiazolyl wherein said heterocycle rings are carbon-nitrogen linked; or

$R_9$ is $(C_1-C_6)$alkyl or $(C_1-C_8)$alkoxy wherein said $(C_1-C_6)$alkyl or $(C_1-C_8)$alkoxy is optionally monosubstituted with cyclo$(C_4-C_7)$alken-1-yl, phenyl, thienyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, thiomorpholinyl, 1-oxothiomorpholinyl, 1,1-dioxothiomorpholinyl, pyridazinyl, pyrimidinyl, pyrazinyl, piperazinyl, 1,3,5-triazinyl or indolyl and wherein said $(C_1-C_6)$alkyl or $(C_1-C_8)$alkoxy are optionally additionally independently mono-or di-substituted with halo, hydroxy, $(C_1-C_5)$alkoxy, amino, mono-N- or di-N,N-$(C_1-C_5)$alkylamino, cyano, carboxy, or $(C_1-C_4)$alkoxycarbonyl; and

wherein the $R_9$ rings are optionally mono- or di-substituted independently on carbon with halo, $(C_1-C_4)$alkyl, $(C_1-C_4)$alkoxy, hydroxy, hydroxy$(C_1-C_4)$alkyl, amino$(C_1-C_4)$alkyl, mono-N- or di-N,N-$(C_1-C_4)$alkylamino$(C_1-C_4)$alkyl, $(C_1-C_4)$alkoxy$(C_1-C_4)$alkyl, amino, mono-N- or di-N,N-$(C_1-C_4)$alkylamino, cyano, carboxy, $(C_1-C_5)$alkoxycarbonyl, carbamoyl, formyl or trifluoromethyl and said $R_9$ rings may optionally be additionally mono- or di-substituted independently with $(C_1-C_5)$alkyl or halo;

with the proviso that no quaternized nitrogen on any $R_9$ heterocycle is included;

$R_{12}$ is morpholino, thiomorpholino, 1-oxothiomorpholino, 1,1-dioxothiomorpholino, thiazolidin-3-yl, 1-oxothiazolidin-3-yl, 1,1-dioxothiazolidin-3-yl, pyrrolidin-1-yl, piperidin-1-yl, piperazin-1-yl, piperazin-4-yl, azetidin-1-yl, 1,2-oxazinan-2-yl, pyrazolidin-1-yl, isoxazolidin-2-yl, isothiazolidin-2-yl, 1,2-oxazetidin-2-yl, oxazolidin-3-yl, 3,4-dihydroisoquinolin-2-yl, 1,3-dihydroisoindol-2-yl, 3,4-dihydro-2H-quinol-1-yl, 2,3-dihydro-benzo[1,4]oxazin-4-yl, 2,3-dihydro-benzo[1,4]-thiazine-4-yl, 3,4-dihydro-2H-quinoxalin-1-yl, 3,4-dihydro-benzo[c][1,2]oxazin-1-yl, 1,4-dihydrobenzo[d][1,2]oxazin-3-yl, 3,4-dihydro-benzo[e][1,2]-oxazin-2-yl, 3H-benzo[d]isoxazol-2-yl, 3H-benzo[c]isoxazol-1-yl or azepan-1-yl,

wherein said $R_{12}$ rings are optionally mono-, di- or tri-substituted independently with halo, $(C_1-C_5)$alkyl, $(C_1-C_5)$alkoxy, hydroxy, amino, mono-N- or di-N,N-$(C_1-C_5)$alkylamino, formyl, carboxy, carbamoyl, mono-N- or di-N,N-$(C_1-C_5)$alkylcarbamoyl, $(C_1-C_6)$alkoxy$(C_1-C_3)$alkoxy, $(C_1-C_5)$alkoxycarbonyl, benzyloxycarbonyl, $(C_1-C_5)$alkoxycarbonyl$(C_1-C_5)$alkyl, $(C_1-C_4)$alkoxycarbonylamino, carboxy$(C_1-C_5)$alkyl, carbamoyl$(C_1-C_5)$alkyl, mono-N- or di-N,N-$(C_1-C_5)$alkylcarbamoyl$(C_1-C_5)$alkyl, hydroxy$(C_1-C_5)$alkyl, $(C_1-C_4)$alkoxy$(C_1-C_4)$alkyl, amino$(C_1-C_4)$alkyl, mono-N- or di-N,N-$(C_1-C_4)$alkylamino$(C_1-C_4)$alkyl, oxo, hydroxyimino or $(C_1-C_6)$alkoxyimino and wherein no more than two substituents are selected from oxo, hydroxyimino or $(C_1-C_6)$alkoxyimino and oxo, hydroxyimino or $(C_1-C_6)$alkoxyimino are on nonaromatic carbon; and

wherein said $R_{12}$ rings are optionally additionally mono- or di-substituted independently with $(C_1-C_5)$alkyl or halo;

with the proviso that when $R_6$ is $(C_1-C_5)$alkoxycarbonyl or benzyloxycarbonyl then $R_1$ is 5-halo, 5-$(C_1-C_4)$alkyl or 5-cyano and $R_4$ is (phenyl)(hydroxy)$(C_1-C_4)$alkyl, (phenyl)($(C_1-C_4)$alkoxy)$(C_1-C_4)$alkyl, hydroxymethyl or Ar$(C_1-C_2)$alkyl, wherein Ar is thien-2- or -3-yl, fur-2-or -3-yl or phenyl wherein said Ar is optionally mono- or di-substituted independently with halo; with the provisos that when $R_4$ is benzyl and $R_5$ is methyl, $R_{12}$ is not 4-hydroxy-piperidin-1-yl or when $R_4$ is benzyl and $R_5$ is methyl $R_6$ is not $C(O)N(CH_3)_2$;

with the proviso that when $R_1$ and $R_{10}$ and $R_{11}$ are H, $R_4$ is not imidazol-4-ylmethyl, 2-phenylethyl or 2-hydroxy-2-phenylethyl;

with the proviso that when both $R_8$ and $R_9$ are n-pentyl, $R_1$ is 5-chloro, 5-bromo, 5-cyano, 5$(C_1-C_5)$alkyl, 5$(C_1-C_5)$alkoxy or trifluoromethyl;

with the proviso that when $R_{12}$ is 3,4-dihydroisoquinol-2-yl, said 3,4-dihydroisoquinol-2-yl is not substituted with carboxy($(C_1-C_4)$alkyl;

with the proviso that when $R_8$ is H and $R_9$ is $(C_1-C_6)$alkyl, $R_9$ is not substituted with carboxy or $(C_1-C_4)$alkoxycarbonyl on the carbon which is attached to the nitrogen atom N of $NHR_9$; and

with the proviso that when $R_6$ is carboxy and $R_1$, $R_{10}$, $R_{11}$ and $R_5$ are all H, then $R_4$ is not benzyl, H, (phenyl)(hydroxy)methyl, methyl, ethyl or n-propyl.

Compounds of formula II are disclosed in published Patent Cooperartion Treat Publication number WO 96/39384, the complete disclosure of which is hereby incorporated by reference.

Another class of compounds able to bind to this site in vivo have the structure:

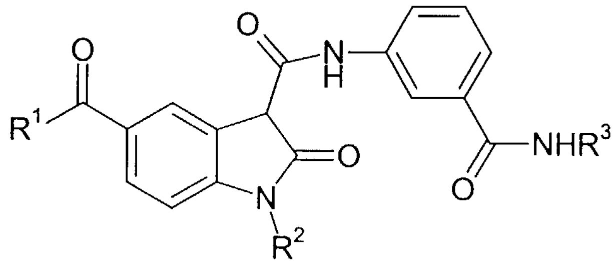

III

a prodrug thereof or a pharmaceutically acceptable salt of said compound or said prodrug wherein:

$R^1$ is $(C_1-C_4)$alkyl, $(C_3-C_7)$cycloalkyl, phenyl or phenyl substituted with up to three $(C_1-C_4)$alkyl, $(C_1-C_4)$alkoxy or halogen;
$R_2$ is $(C_1-C_4)$alkyl; and
$R_3$ is $(C_3-C_7)$cycloalkyl; phenyl; phenyl substituted at the para position with $(C_1-C_4)$alkyl, halo, hydroxy$(C_1-C_4)$alkyl or trifluoromethyl; phenyl substituted at the meta position with fluoro; or phenyl substituted at the ortho position with fluoro.

Compounds of formula III may be synthesised by the following methods

Preparation 1

5-Acetyl-1-ethyl-2-oxo-2,3-dihydro-1H-indole-3-carboxylic acid (3-phenylcarbamoylphenyl)-amide

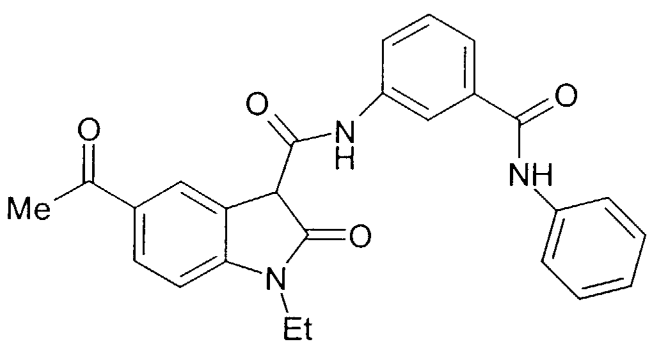

Step A.

1 -Ethyl-5-(2-methyl-[1,3]dioxolan-2-yl)-2-oxo-2,3-dihydro-1H-indole-3-carboxylic acid (3-phenylcarbamoyl-phenyl)-amide.

1 -Ethyl-5-(2-methyl-[1,3]dioxolan-2-yl)-2-oxo-2,3-dihydro-1H-indole-3-carboxylic acid methyl ester (3.6 g, 11.8 mmol) and 3-amino-N-phenyl-benzamide (5.0 g, 24 mmol) were combined in benzene (160 mL) in a flask fitted with a Soxhlet containing activated 4Å molecular sieves. The mixture was heated to reflux for 30 min, then cooled, washed with 0.1 N HCl (2 X 30 mL), water and brine, dried over magnesium sulfate and concentrated in vacuo to a dark orange foam which was purified by flash-chromatography (chloroform / methanol, 20:1) to give a slightly orange foamy solid (3.75 g, 66%)

Step B.

5-Acetyl-1-ethyl-2-oxo-2,3-dihydro-1H-indole-3-carboxylic acid (3-phenylcarbamoyl-phenyl)-amide.

To a solution of 1-ethyl-5-(2-methyl-[1,3]dioxolan-2-yl)-2-oxo-2,3-dihydro-1H-indole-3-carboxylic acid (3-phenylcarbamoyl-phenyl)-amide (the title compound of Preparation 1, Step A, 3.75 g, 7.8 mmol) in THF (70 mL) was added 2 N HCl (20 mL). The mixture was stirred at room temperature for 1 hour then poured into water (200 mL) and extracted with ethyl acetate. The extracts were washed with water and brine, dried over magnesium sulfate and concentrated in vacuo to afford an orange foam. The foam was dissolved in a small amount of ethyl acetate and precipitated with hexane to yield a tan solid, which was dried on high vacuum (mp 173-175 °C, 2.5 g, 78%). Calculated for $C_{26}H_{23}N_3O_4$: C 70.74; H 5.25; N 9.52; found C70.91; H 5.56; N 9.25.

Preparations 2-15

Preparations 2 to 15 were prepared from the appropriate starting materials in a manner analogous to the method of Preparation 1, with variations in reaction time, temperature, and reagents as noted.

Preparation 2

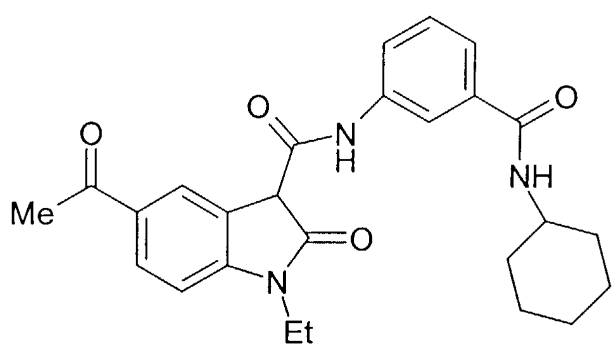

5-Acetyl-1-ethyl-2-oxo-2,3-dihydro-1H-indole-3-carboxylic acid (3-cyclohexylcarbamoyl-phenyl)-amide.

Prepared as in Preparation 1 from 1-ethyl-5-(2-methyl-[1,3]dioxolan-2-yl)-2-oxo-2,3-dihydro-1H-indole-3-carboxylic acid methyl ester and 3-amino-N-cyclohexyl-benzamide. Purified by trituration in ethyl acetate. mp 180°C dec.

Preparation 3

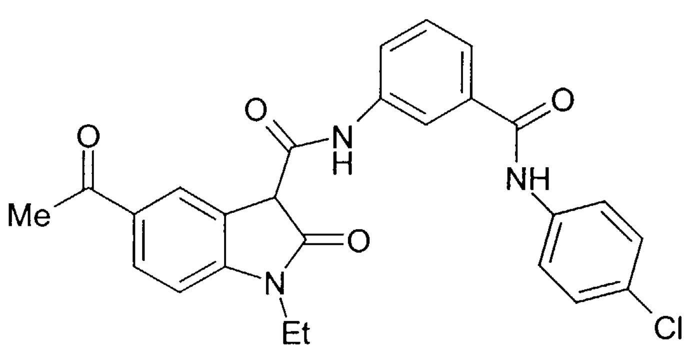

5-Acetyl-1-ethyl-2-oxo-2,3-dihydro-1H-indole-3-carboxylic acid [3-(4-chlorophenylcarbamoyl)-phenyl]-amide.

Prepared as in Preparation 1 from 1-ethyl-5-(2-methyl-[1,3]dioxolan-2-yl)-2-oxo-2,3-dihydro-1H-indole-3-carboxylic acid methyl ester and 3-amino-N-(4-chloro-phenyl)-benzamide. Recristallized from ethyl acetate - hexanes. mp 119-121°C

Preparation 4

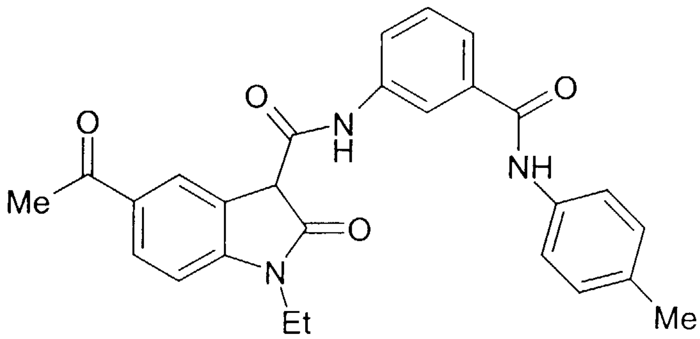

5-Acetyl-1-ethyl-2-oxo-2,3-dihydro-1H-indole-3-carboxylic acid (3-p-tolylcarbamoylphenyl)-amide.

Prepared as in Preparation 1 from 1-ethyl-5-(2-methyl-[1,3]dioxolan-2-yl)-2-oxo-2,3-dihydro-1H-indole-3-carboxylic acid methyl ester and 3-amino-N-(p-tolyl)-benzamide. Purified by trituration in ethyl acetate / hexanes. mp 168 dec.

Preparation 5

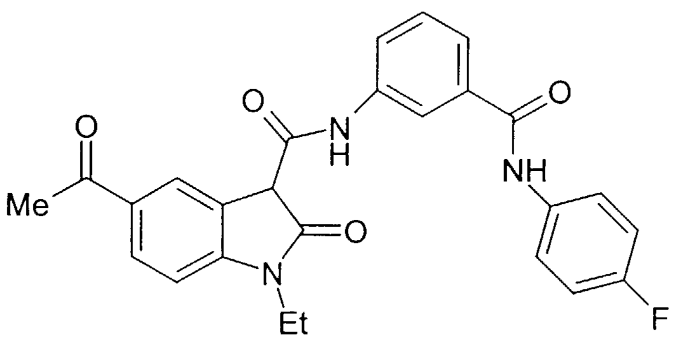

5-Acetyl-1-ethyl-2-oxo-2,3-dihydro-1H-indole-3-carboxylic acid [3-(4-fluorophenylcarbamoyl)-phenyl]-amide.

Prepared as in Preparation 1 from 1-ethyl-5-(2-methyl[1,3]dioxolan-2-yl)-2-oxo-2,3-dihydro-1H-indole-3-carboxylic acid methyl ester and 3-amino-N-(4-fluoro-phenyl)-benzamide. Recrystallized from ethyl acetate - hexanes - benzene. Calculated for $C_{26}H_{22}FN_3O_4$: C 67.97; H 4.83; N 9.15; found C 68.34; H 5.31; N 8.80.

Preparation 6

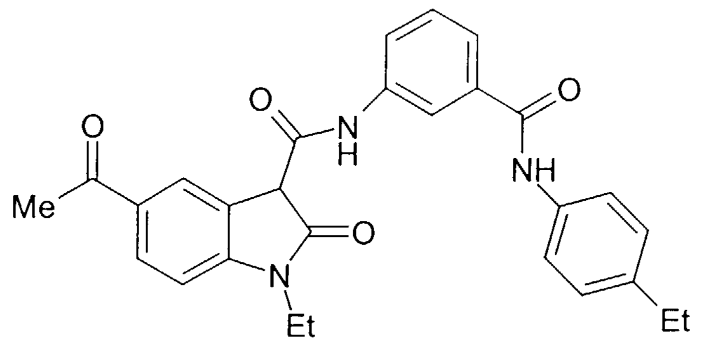

5-Acetyl-1-ethyl-2-oxo-2,3-dihydro-1H-indole-3-carboxylic acid [3-(4-ethylphenylcarbamoyl)-phenyl]-amide.

Prepared as in Preparation 1 from 1-ethyl-5-(2-methyl-[1,3]dioxolan-2-yl)-2-oxo-2,3-dihydro-1H-indole-3-carboxylic acid methyl ester and 3-amino-N-(4-ethyl-phenyl)-benzamide. Purified by chromatography (chloroform / methanol, 10:1). $^1$H NMR (DMSO-d6) δ 1.15 (m, 6 H), 2.5 (q, 2 H), 3.85 (q, 2 H), 6.85-8.3 (m, 11 H), 10.0 (s, 1 H), 11.1 (s, 1 H).

Preparation 7

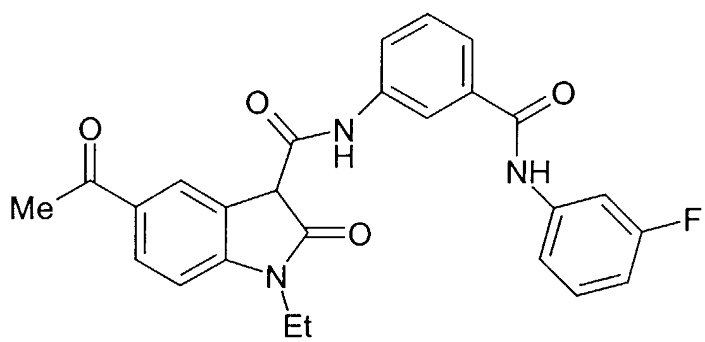

5-Acetyl-1-ethyl-2-oxo-2,3-dihydro-1H-indole-3-carboxylic acid [3-(3-fluorophenylcarbamoyl)-phenyl]-amide.

Prepared as in Preparation 1 from 1-ethyl-5-(2-methyl-[1,3]dioxolan-2-yl)-2-oxo-2,3-dihydro-1H-indole-3-carboxylic acid methyl ester and 3-amino-N-(3-fluoro-phenyl)-benzamide. $^1$H NMR ($CDCl_3$) δ 1.25 (t, 3 H), 2.6 (s, 3 H), 3.85 (q, 2 H), 4.45 (s, 1 H), 6.85-8.05 (m, 11 H), 8.3 (s, 1 H), 9.65 (s, 1 H).

Preparation 8

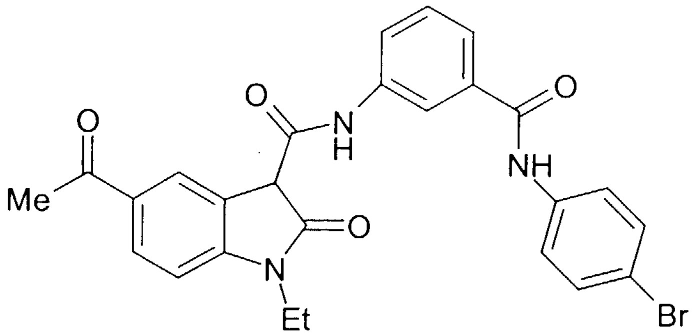

5-Acetyl-1-ethyl-2-oxo-2,3-dihydro-1H-indole-3-carboxylic acid [3-(4-bromophenylcarbamoyl)-phenyl]-amide.

Prepared as in Preparation 1 from 1-ethyl-5-(2-methyl[1 ,3]dioxolan-2-yl)-2-oxo-2,3-dihydro-1H-indole-3-carboxylic acid methyl ester and 3-amino-N-(4-bromo-phenyl)-benzamide. mp 117-120°C.

Preparation 9

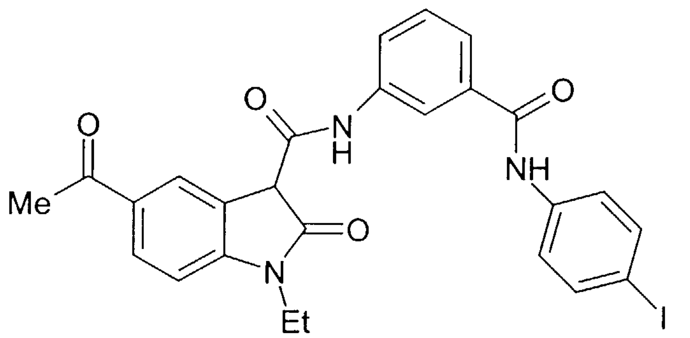

5-Acetyl-1-ethyl-2-oxo-2,3-dihydro-1H-indole-3-carboxylic acid [3-(4-iodophenylcarbamoyl)-phenyl]-amide.

Prepared as in Preparation 1 from 1-ethyl-5-(2-methyl-[1,3]dioxolan-2-yl)-2-oxo-2,3-dihydro-1H-indole-3-carboxylic acid methyl ester and 3-amino-N-(4-iodo-phenyl)-benzamide. mp 205-210 °C. Calculated for $C_{26}H_{22}IN_3O_4$: C 55.04; H 3.91; N 7.41; found C 55.13; H 4.04; N 7.12.

Preparation 10

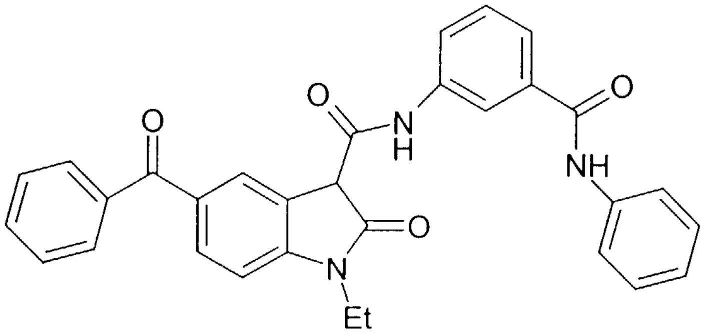

5-Benzoyl-1-ethyl-2-oxo-2,3-dihydro-1H-indole-3-carboxylic acid (3-phenylcarbamoylphenyl)-amide.

Prepared as in Preparation 1, Step A, from 5-benzoyl-1-ethyl-2-oxo-2,3-dihydro-1H-indole-3-carboxylic acid ethyl ester (US 4,686,224) and 3-amino-N-phenyl-benzamide. Purified by trituration in ethyl acetate / hexanes. mp 129-135 °C.

Preparation 11

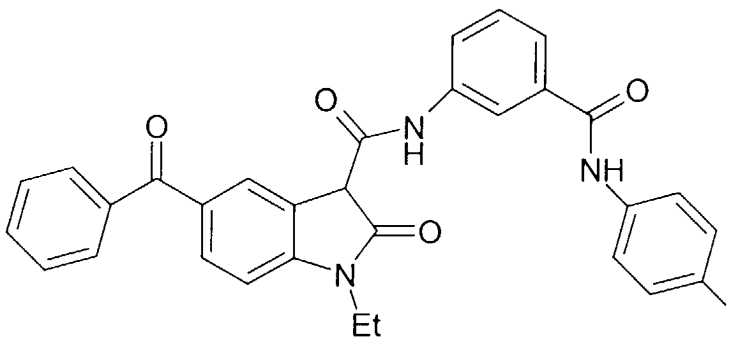

5-Benzoyl-1-ethyl-2-oxo-2,3-dihydro-1H-indole-3-carboxylic acid [3-(4-bromophenylcarbamoyl)-phenyl]-amide.

Prepared as in Preparation 1, Step A, from 5-benzoyl-1-ethyl-2-oxo-2,3-dihydro-1H-indole-3-carboxylic acid ethyl ester (US 4,686,224) and 3-amino-N-(4-bromo-phenyl)-benzamide. Purified by flash-chromatography (hexanes / acetone, 1:1) followed by trituration in ethyl acetate / hexanes. mp 139-142 °C.

Preparation 12

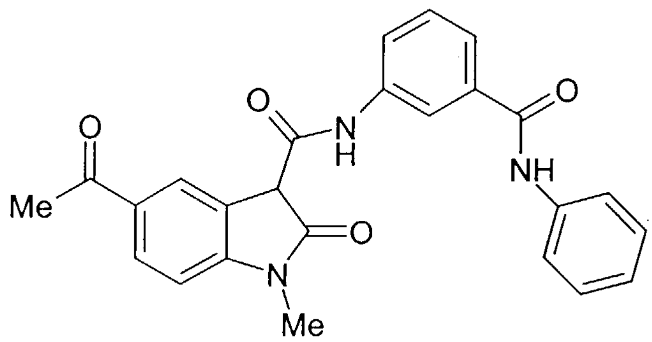

5-Acetyl-1-methyl-2-oxo-2,3-dihydro-1H-indole-3-carboxylic acid (3-phenylcarbamoylphenyl)-amide.

Prepared as in Preparation 1 from 1-methyl-5-(2-methyl-[1,3]dioxolan-2-yl)-2-oxo-2,3-dihydro-1H-indole-3-carboxylic acid methyl ester and 3-amino-N- phenyl-benzamide. Purified by trituration in hexanes / ethyl acetate. mp 178-179 °C. Calculated for $C_{25} H_{21} N_3 O_4$: C 70.25; H 4.95; N 9.83; found C 69.89; H 4.79; N 9.69.

Preparation 13

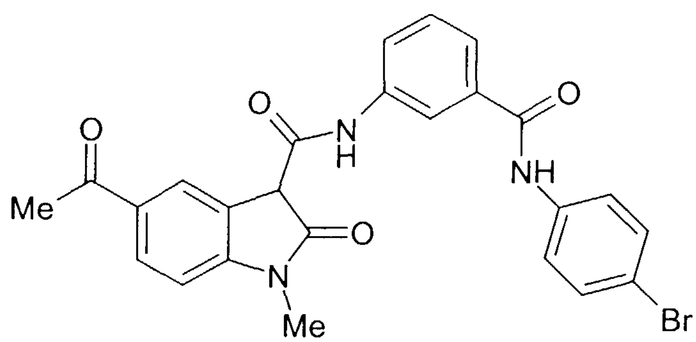

5-Acetyl-1-methyl-2-oxo-2,3-dihydro-1H-indole-3-carboxylic acid [3-(4-bromophenylcarbamoyl)-phenyl]-amide.

Prepared as in Preparation 1 from 1-methyl-5-(2-methyl[1 ,3]dioxolan-2-yl)-2-oxo-2,3-dihydro-1H-indole-3-carboxylic acid methyl ester and 3-amino-N-(4-bromo-phenyl)-benzamide. Purified by flash-chromatography (hexanes / acetone, 1:1).

mp 234-236 °C. Calculated for $C_{25}H_{20}BrN_3N_3O_4$: C 59.30; H 3.98; N 8.30; found C 59.12; H 4.15; N 8.08.

Preparation 14

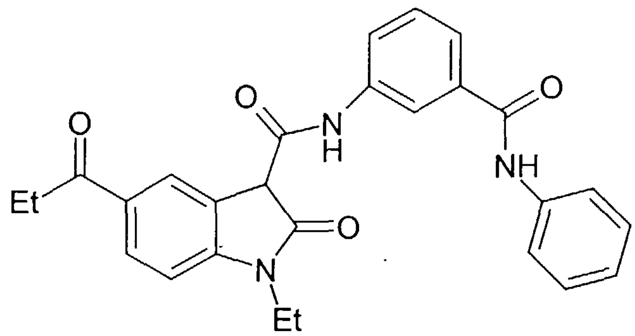

1 -Ethyl-2-oxo-5-propionyl-2,3-dihydro-1H-indole-3-carboxylic acid (3-phenylcarbamoyl-phenyl)-amide.

Prepared as in Preparation 1 from 1-ethyl-5-(2-ethyl-[1,3]dioxolan-2-yl)-2-oxo-2,3-dihydro-1H-indole-3-carboxylic acid methyl ester and 3-amino-N-phenyl-benzamide. Purified by flash-chromatography (chloroform / methanol, 20:1) followed by trituration in hexanes / ethyl acetate. mp 179-180 °C. Calculated for $C_{27}H_{25}N_3O_4$: C 71.19; H 5.53; N 9.22; found C 70.79; H 5.73; N 8.79.

Preparation 15

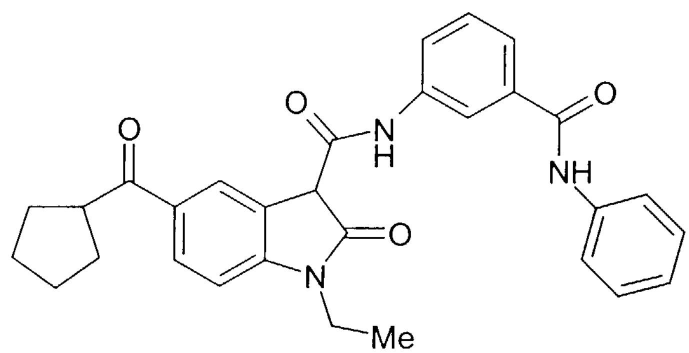

5-Cyclopentanecarbonyl-1-ethyl-2-oxo-2,3-dihydro-1H-indole-3-carboxylic acid (3-phenylcarbamoyl-phenyl)-amide.

Prepared as in Preparation 1 from 5-(2-cyclopentyl-[1,3]dioxolan-2-yl)-1-ethyl-2-oxo-2,3-dihydro-1H-indole-3-carboxylic acid ethyl ester and 3-amino-N-phenyl-benzamide. Purified by flash-chromatography (hexanes / acetone, 1:1) followed by trituration in ethyl acetate. mp 208-213 °C.

Preparation 16

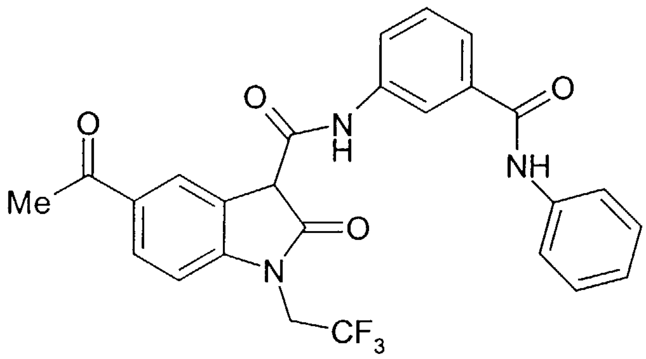

5-Acetyl-2-oxo-1-(2,2,2-trifluoro-ethyl)-2,3-dihydro-1H-indole-3-carboxylic acid (3-phenylcarbamoyl-phenyl)-amide.

Sodium hydride (0.13 g of a 60% suspension, 3.3 mmol) was added to a solution of 5-(2-methyl-[1,3]dioxolan-2-yl)-1-(2,2,2-trifluoro-ethyl)-1,3-dihydro-indol-2-one (0.5 g, 1.66 mmol) in HMPA (6 mL) and after 10 min 3-(3,3-diphenyl-ureido)-N-phenyl-benzamide (0.74 g, 1.8 mmol) was added. The reaction mixture was stirred for three days, acidified with 1 N HCl, poured into water and extracted with ethyl acetate. The combined extracts were washed with water and brine, dried over magnesium sulfate and concentrated in vacuo to a dark foam. Trituration in ethyl acetate / hexanes gave the title compound as a colorless solid (124 mg, 15%, mp 197-203 °C). Calculated for $C_{26} H_{20} F_3 N_3 O_4$: C 63.03; H 4.07; N 8.48; found C 62.83; H 4.32; N 8.47.

Preparation 17

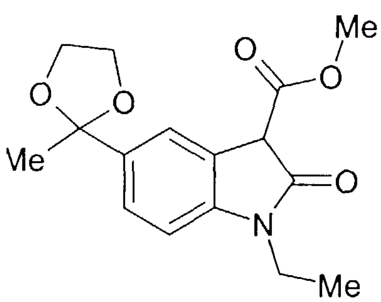

1-Ethyl-5-(2-methyl-[1,3]dioxolan-2-yl)-2-oxo-2,3-dihydro-1H-indole-3-carboxylic acid methyl ester.

Sodium (2.68 g, 112 mmol) was added to methanol (110 mL) in a 3-neck flask fitted with a reflux condenser, while controlling the temperature with an ice-bath. After dissolution dimethyl carbonate (9.4 mL, 112 mmol) was added followed by 1-ethyl-5-(2-methyl-[1,3]dioxolan-2-yl)-1,3-dihydro-indol-2-one (US 4,686,224, 9.2 g, 37 mmol). The mixture was heated to reflux for 70 hours, then cooled, concentrated to about 25 mL, diluted with water, acidified to pH 7 with acetic acid and extracted twice with ethyl acetate. The combined extracts were washed with brine, dried over magnesium sulfate and concentrated in vacuo to an oily solid which was triturated in isopropyl ether, collected and dried (7.9 g, 70%).

Preparations 18-20

The compounds of Preparations 18 to 20 were prepared from the appropriate starting materials in a manner analogous to the method of Preparation 1.

Preparation 18

1-Methyl-5-(2-methyl-[1,3]dioxolan-2-yl)-2-oxo-2,3-dihydro-1H-indole-3-carboxylic acid methyl ester.

Prepared from 1-methyl-5-(2-methyl-[1,3]dioxolan-2-yl)-2,3-dihydro-indol-2-one, which was prepared as disclosed in U.S. 4,686,224.

Preparation 19

1 -Ethyl-5-(2-ethyl-[1,3]dioxolan-2-yl)-2-oxo-2,3-dihydro-1H-indole-3-carboxylic acid methyl ester.

Prepared from 1-ethyl-5-(2-ethyl-[1,3]dioxolan-2-yl)-2,3-dihydro-indol-2-one, the title compound of Preparation 38.

Preparation 20

(2-Cyclopentyl-[1,3]dioxolan-2-yl)-1-ethyl-2-oxo-2,3-dihydro-1H-indole-3-carboxylic acid ethylester.

Prepared from 5-(2-cyclopentyl-[1,3]dioxolan-2-yl)-1-ethyl-2,3-dihydro-indol-2-one, the title compound of Preparation 40.

Preparation 21

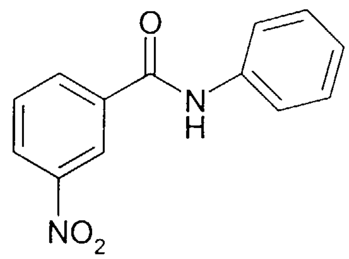

3-Nitro-N-phenyl-benzamide.

To a cooled (0°C) solution of aniline (17 g, 180 mmol) and triethylamine (27 mL, 190 mmol) in dichloromethane (100 mL) was added a solution of 3-nitrobenzoyl chloride (30 g, 160 mmol) in dichloromethane (60 mL). The mixture was stirred for 15 min at 0°C then overnight at room temperature. It was then poured into saturated sodium bicarbonate (1 L) and stirred vigorously for 15 min. The precipitate was collected, washed with water and dried (40 g, 100%).

Preparation 22

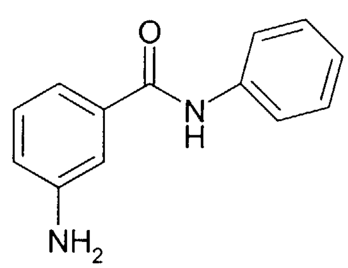

3-Amino-N-phenyl-benzamide.

10% Palladium on carbon (2.0 g) was added to a solution of 3-nitro-N-phenyl-benzamide (20 g, 83 mmol) in ethanol (225 mL) and the mixture was hydrogenated at 45 psi for 2 hours. The mixture was filtered through diatomaceous earth and concentrated to give a colorless solid (mp 118-120 °C, 16 g, 90%).

Preparations 23-31

The compounds of Preparations 23 to 31 were prepared from the appropriate commercially available starting materials in a manner analogous to the methods of Preparations 21 and 22 performed sequentially.

Preparation 23

3-Amino-N-cyclohexyl-benzamide.

Preparation 24

3-Amino-N-(4-chloro-phenyl)-benzamide.

Preparation 25

3-Amino-N-(p-tolyl)-benzamide.

Preparation 26

3-Amino-N-(4-fluoro-phenyl)-benzamide.

Preparation 27

3-Amino-N-(4-ethyl-phenyl)-benzamide.

Preparation 28

3-Amino-N-(3-fluoro-phenyl)-benzamide.

Preparation 29

3-Amino-N-(4-bromo-phenyl)-benzamide.

Preparation 30

3-Amino-N-(4-iodo-phenyl)-benzamide.

Preparation 31

3-Amino-N-(3-methyl-phenyl)-benzamide.

Preparation 32

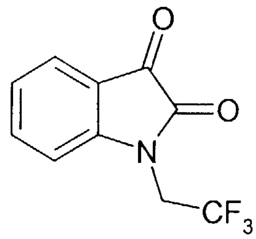

(2,2,2-Trifluoro-ethyl)-1H-indole-2,3-dione.

Sodium hydride (8.65 g of a 60 % oil dispersion, 0.22 mol) was washed with hexane and suspended in hexamethylphosphoramide (200 mL). Isatin (29.4 g, 0.20 mol)) was added carefully. After the gas evolution subsided, 2,2,2-trifluoroethyl iodide (46.2 g, 0.22 mol) was added and the mixture was heated to 55 °C for 4 hours. The solution was cooled, diluted with water (1 L), and the precipitate was collected. The filtrate was acidified with 6 N HCl and a new precipitate formed and was collected. The combined solids were recrystallized from 95% ethanol. Yield 22.5 g (49%). mp 161-163 °C.

Preparation 33

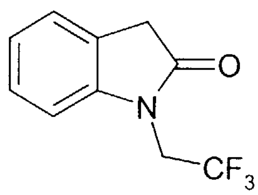

(2,2,2-Trifluoro-ethyl)-1,3-dihydro-indol-2-one.

A mixture of 1-(2,2,2-trifluoro-ethyl)-1H-indole-2,3-dione (9.2 g, 40 mmol) and 10% palladium on carbon (2.4 g) in acetic acid (100 mL) and 70% perchloric acid (6.4 mL, 80 mmol) was hydrogenated in a Parr apparatus for 22 hours. The mixture was filtered through diatomaceous earth, diluted with water (1.5 L) and the precipitate was collected and dried. mp 155-162 °C. Yield 7.5 g (87%).

Preparation 34

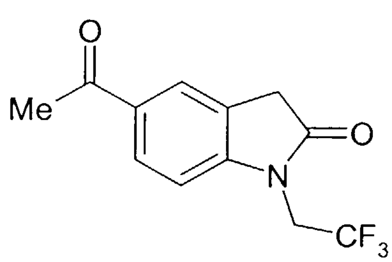

5-Acetyl-1-(2,2,2-trifluoro-ethyl)-1,3-dihydro-indol-2-one.

To a solution of 1-(2,2,2-trifluoro-ethyl)-1,3-dihydro-indol-2-one (2.0 g, 9.3 mmol) and acetyl chloride (0.86

mL, 12 mmol) in carbon disulfide (40 mL) was added aluminum trichloride (7.4 g, 56 mmol) by portions. The mixture was heated to reflux for 3 hours, then cooled. The liquid phase was decanted and the residue was quenched carefully with ice, then water. The solids were filtered, washed with water, dried and recrystallized from acetone / hexanes to give a pale pink solid, mp 170-171 °C. Yield 1.23 g (51%).

Preparation 35

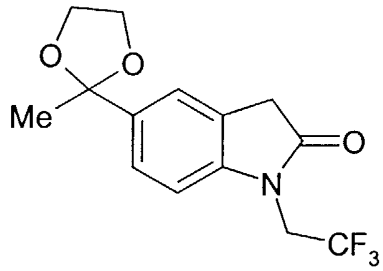

(2-Methyl-[1,3]dioxolan-2-yl)-1-(2,2,2-trifluoro-ethyl)-1,3-dihydro-indol-2-one.

A solution of 5-acetyl-1-(2,2,2-trifluoro-ethyl)-1,3-dihydro-indol-2-one (1.04 g, 4.0 mmol), ethylene glycol (1.37 mL, 24 mmol) and p-toluenesulfonic acid (10 mg) in benzene (25 mL) was heated to reflux for 4 hours in a flask fitted with a Dean-Stark trap. The solution was diluted with ethyl acetate, washed with saturated sodium bicarbonate, water and brine, dried over magnesium sulfate and concentrated in vacuo to an oil which solidified upon standing (1.19 g, 98%, mp 87-89 °C).

Preparation 36

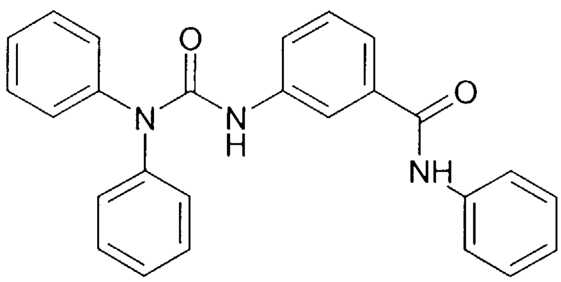

(3,3-Diphenyl-ureido)-N-phenyl-benzamide.

A mixture of 3-amino-N-phenyl-benzamide (2.0 g, 9.4 mmol), diphenylcarbamoyl chloride (2.2 g, 9.4 mmol) and triethylamine (2.6 mL, 19 mmol) in ethanol (10 mL) was heated to reflux for 4.5 hours. The mixture was cooled and concentrated, water was added, the slurry was acidified with 1 N HCl and the solid was collected, washed with water, dried and recrystallized from acetone / hexanes, to give a colorless solid (1.63 g, 42%).

Preparation 37

1-Ethyl-5-propionyl-2,3-dihydro-indol-2-one.

Prepared from readily available starting materials (N-ethyloxindole and propionyl chloride) in a manner analogous to that set forth in Preparation 34.

Preparation 38

1-Ethyl-5-(2-ethyl-[1,3]dioxolan-2-yl)-2,3-dihydro-indol-2-one.

Prepared from the title compound of Preparation 35 in a manner analogous to that set forth in Preparation 19.

Preparation 39

5-Cyclopentanecarbonyl-1-ethyl-2,3-dihydro-indol-2-one.

Prepared from readily available starting materials (N-ethyloxindole and cyclopentanecarbonyl chloride) in a manner analogous to that set forth in Preparation 34.

Preparation 40

(2-cyclopentyl-[1,3]dioxolan-2-yl)-1-ethyl-2,3-dihydro-indol-2-one.

Prepared from the title compound of Preparation 39 in a manner analogous to that set forth in Preparation 35.

**General Experimental Procedures.** NMR spectra were recorded on a Bruker AM-300 spectrometer at about 22 °C. Chemical shifts are expressed in parts per million downfield from trimethylsilane. Thermospray MS (TSPMS) were obtained on a Fisons Trio-1000 spectrometer using ammonia ionization. Chemical ionization mass spectra (PBMS) were obtained either on a Hewlett-Packard 5989 instrument (ammonia ionization). Silica gel chromatography was performed with 30µM, 60Å pore size silica or equivalent in glass columns under low nitrogen pressure. Columns were packed wet in and eluted with an appropriate composition of the specified solvent or solvent mixture or a gradient where the proportion of the polar solvent was increased until the target substance eluted from the column.

Preparation 41

Azetidine-3-carboxylic acid methyl ester hydrochloride.

A mixture of azetidine-3-carboxylic acid (1.26 g) and chlorotrimethylsilane (7.1 mL) in methanol (10 mL) was heated at reflux for 5 h and concentrated leaving a solid (1.88 g, 100%). $^{1}H$ NMR (300 mHz, $D_2O$) d 4.31 (m, 4H), 3.81 (m, 1H), 3.78 (s, 3H).

Preparation 42

(S)-1-(2-tert-Butoxycarbonylamino-3-phenyl-propionyl)-azetidine-3-carboxylic acid methyl ester.

1-(3-Dimethylaminopropyl)3-ethylcarbodiimide hydrochloride (2.26 g, 11.8 mmol) was added to a mixture of azetidine-3-carboxylic acid methyl ester hydrochloride (1.79 g, 11.8 mmol), N-t-butoxycarbonyl-L-phenylalanine (3.13 g, 11.8 mmol), triethylamine (1.20 g, 11.8 mmol), and 1-hydroxybenzotriazole hydrate (2.72 g, 17.7 mmol) in dichloromethane (25 mL) at 0 °C, and the mixture was allowed to warm to 20 °C over several hours and stirred at room temperature for 72h. The mixture was diluted with ethyl acetate and the resulting mixture washed three times with 1N NaOH, three times with 1N HCI, once with aqueous NaHCO3, dried and concentrated giving 2.8 g of the title product. $^{1}H$ NMR (300 mHz, $CDCl_3$) d 7.35-7.2 (m, 5H), 5.30 (m, 1H), 4.35-3.95 (m, 4H), 3.71 (s, 3H), 3.3-3.15 (m, 1H), 3.1-2.8 (m, 3H), 1.49 (s, 4.5H), 1.47 (s, 4.5H). PBMS 363 (MH+).

Preparation 43

(*S*)-1-(2-Amino-3-phenyl-propionyl)-azetidine-3-carboxylic acid methyl ester hydrochloride.

(*S*)-1-(2-tert-Butoxycarbonylamino-3-phenyl-propionyl)-azetidine-3-carboxylic acid methyl ester (2.74 g, 7.6 mmol) was dissolved in 4M HCI-dioxanes (28 mL) at 0 °C. The resulting mixture was stirred at 25 °C for 1h, concentrated, and the residue ground up under ether and filtered. The colorless solid was collected and dried (2.17 g, 96%).

Preparation 44

(S)-1-{2-[(5-Chloro-1H-indole-2-carbonyl)-amino]-3-phenyl-propionyl}-azetidine-3-carboxylic acid methyl ester.

1-(3-Dimethylaminopropyl)3-ethylcarbodiimide hydrochloride (1.15 g, 6.0 mmol) was added to a mixture of (S)-1-(2-Amino-3-phenyl-propionyl)-azetidine-3-carboxylic acid methyl ester hydrochloride (1.8 g, 6.0 mmol), triethylamine (610 mg, 6.0 mmol), 5-chloro-1H-indole-2-carboxylic acid ( 1.2 g, 6.0 mmol) and 1-hydroxybenzotriazole hydrate (1.39 g, 9.0 mmol) in dichloromethane (18 mL) at 0 °C, and the mixture was allowed to warm to 20 °C over several hours and stirred at room temperature for 72h. The mixture was diluted with ethyl acetate and the resulting mixture washed three times with 1N NaOH, three times with 1N HCl, once with aqueous NaHCO3, dried and concentrated giving 2.8 g of a yellow foam, which was purified by chromatography on silica gel eluting with a gradient of ethyl acetate in hexanes, followed by ethyl acetate giving a colorless foam ( 2.24 g, 86%). $^1$H NMR (300 mHz, $CDCl_3$) d 9.28 (d, 1H, J = 9.2 Hz), 7.59 (m, 1H), 7.32-7.19 (m, 10H), 6.83 (m, 1H), 4.76 (m, 1H), 4.3-4.05 (m, 3H), 3.71 (s, 1.5H), 3.69 (s, 1.5H), 3.33 (m, 1H), 3.3-3.0 (m, 3H). TSPMS 440 (MH+, 100%)

Preparation 45

(S)-1-{2-[(5-Chloro-1H-indole-2-carbonyl)-aminol-3-phenyl-propionyl}-azetidine-3-carboxylic acid sodium salt.

A solution of 1-{2-[(5-chloro-1H-indole-2-carbonyl)-amino]-3-phenyl-propionyl}-azetidine-3-carboxylic acid methyl ester (150 mg, 0.34 mmol) in methanol (1 mL) was treated at 23°C with 1.0 N aqueous NaOH (0.34 mL). After 30 min the mixture was concentrated in vacuo and the residue triturated with ether and dried (141 mg, 93%). $^1$H NMR (300 mHz, $D_2O$) d 7.61 (m, 1H), 7.41-7.22 (m, 9H), 6.98 (m, 1H), 4.65-4.52 (m, 1H), 4.4-4.2 (m, 1H), 3.86 (m, 0.5H), 3.64 (m, 0.5H), 3.35-3.2 (m, 1H), 3.2-3.0 (m, 3H). TSPMS 426 (MH+, 100%). HPLC (1:1 MeCN-pH 2.1 0.1M KH2PO4-H3PO4, 1.0 mLmin, 4.6 x 250 mm Rainin Microsorb™ C-18, 214 nM) 4.43 min (96%). Anal. Calcd for $C_{22}H_{19}ClN_3O_4Na$+3.5$H_2O$: C, 51.72; H, 5.12; N, 8.22. Found: C, 51.49; H, 5.02; N, 8.01.

Preparation 46

(S)-1-{2-[(5-Chloro-1H-indole-2-carbonyl)-amino]-3-phenyl-propionyl}-azetidine-3-carboxylic acid.

The preceding corresponding sodium salt was partitioned between ethyl acetate and aqueous 2N HCl. The aqueous layer was separated and extracted with ethyl acetate. The ethyl acetate layers were combined, dried over MgSO4, and concentrated, giving the free acid in quantitative yield. $^1$H NMR (300 mHz, DMSO-*d*6, partial) d 11.78 (s, 0.5H), 11.76 (s, 0.5H), 8.9 (m, 1H), 7.72 (m, 1H), 7.45-7.15 (m,8H), 4.65 (m, 1H), 4.45 (t, 0.5H), 4.37 (m, 0.5H), 4.15 (m, 0.5H), 4.1-3.8 (m, 2.5H), 3.1-2.98 (m, 2H).

The compounds of this invention are readily adapted to clinical use as hypoglycemic agents. The hypoglycemic activity of the compounds of this invention can be determined by the amount of test compound that reduces glucose levels relative to a vehicle without test compound in male *ob*/*ob* mice. The test also allows the determination of an approximate minimal effective dose (MED) value for the *in vivo* reduction of plasma glucose concentration in such mice for such test compounds.

Since the concentration of glucose in blood is closely related to the development of diabetic disorders, the compounds of this invention, by virtue of their hypoglycemic action, prevent, arrest and/or regress diabetic disorders.

Five to eight week old male C57BL/6J-*ob*/*ob* mice (obtained from Jackson Laboratory, Bar Harbor, ME) are housed five per cage under standard animal care practices. After a one week acclimation period, the animals are weighed and 25 microliters of blood are collected from the retro-orbital sinus prior to any treatment. The blood sample is immediately diluted 1:5 with saline containing 0.025% sodium heparin, and held on ice for metabolite analysis. Animals are assigned to treatment groups so that each group has a similar mean for plasma glucose concentration. After group assignment, animals are dosed orally each day for four days with the vehicle consisting of either: (1) 0.25% w/v methyl cellulose in water without pH adjustment; or (2) 0.1% Pluronic® P105 Block Copolymer Surfactant (BASF Corporation, Parsippany, NJ) in 0.1% saline without pH adjustment. On day 5, the animals are weighed again and then dosed orally with a test compound or the vehicle alone. All drugs are administered in vehicle consisting of either: (1) 0.25% w/v methyl cellulose in water without pH adjustment; or (2) 10% DMSO/0.1% Pluronic® in 0.1% saline without pH adjustment. The animals are then bled from the retro-orbital sinus three hours later for determination of blood metabolite levels. The freshly collected samples are centrifuged for two minutes at 10,000 x g at room temperature. The supernatant is analyzed for glucose, for example, by the Abbott VP™ (Abbott Laboratories, Diagnostics Division, Irving, TX) and VP Super System® Autoanalyzer (Abbott Laboratories, Irving, TX) or the Abbott Spectrum CCX™ (Abbott Laboratories, Irving, TX), using the A-Gent™Glucose-UV Test reagent system (Abbott Laboratories, Irving,

TX) (a modification of the method of Richterich and Dauwalder, Schweizerische Medizinische Wochenschrift, 101, 860 (1971)) (hexokinase method) using a 100 mg/dL standard. Plasma glucose is then calculated by the equation:

Plasma glucose (mg/dL)=Sample value x 8.14

where 8.14 is the dilution factor, adjusted for plasma hematocrit (assuming the hematocrit is 44%).

The animals dosed with vehicle maintain substantially unchanged hyperglycemic glucose levels (e.g., greater than or equal to 250 mg/dL), animals treated with compounds having hypoglycemic activity at suitable doses have significantly depressed glucose levels. Hypoglycemic activity of the test compounds is determined by statistical analysis (unpaired t-test) of the mean plasma glucose concentration between the test compound group and vehicle-treated group on day 5. The above assay carried out with a range of doses of test compounds allows the determination of an approximate minimal effective dose (MED) value for the *in vivo* reduction of plasma glucose concentration.

The compounds of this invention are readily adapted to clinical use as hyperinsulinemia reversing agents, triglyceride lowering agents and hypocholesterolemic agents. Such activity can be determined by the amount of test compound that reduces insulin, triglycerides or cholesterol levels relative to a control vehicle without test compound in male *ob*/*ob* mice.

Since the concentration of cholesterol in blood is closely related to the development of cardiovascular, cerebral vascular or peripheral vascular disorders, the compounds of this invention by virtue of their hypocholesterolemic action, prevent, arrest and/or regress atherosclerosis.

Since the concentration of insulin in blood is related to the promotion of vascular cell growth and increased renal sodium retention, (in addition to the other actions, e.g., promotion of glucose utilization) and these functions are known causes of hypertension, the compounds of this invention by virtue of their hypoinsulinemic action, prevent, arrest and/or regress hypertension.

Since the concentration of triglycerides and/or free fatty acids in blood contributes to the overall levels of blood lipids, the compounds of this invention by virtue of their triglyceride lowering and/or free fatty acid lowering activity prevent, arrest and/or regress hyperlipidemia.

Free fatty acids contribute to the overall level of blood lipids and independently have been negatively correlated with insulin sensitivity in a variety of physiologic and pathologic states.

Five to eight week old male C57BL/6J-*ob*/*ob* mice (obtained from Jackson Laboratory, Bar Harbor, ME) are housed five per cage under standard animal care practices and fed standard rodent diet ad libitum. After a one week acclimation period, the animals are weighed and 25 microliters of blood are collected from the retro-orbital sinus prior to any treatment. The blood sample is immediately diluted 1:5 with saline containing 0.025% sodium heparin, and held on ice for plasma glucose analysis. Animals are assigned to treatment groups so that each group has a similar mean for plasma glucose concentration. The compound to be tested is administered by oral gavage as an about 0.02% to 2.0% solution (weight/volume (w/v)) in either (1) 10% DMSO/0.1% Pluronic® P105 Block Copolymer Surfactant (BASF Corporation, Parsippany, NJ) in 0.1% saline without pH adjustment or (2) 0.25% w/v methylcellulose in water without pH adjustment. Single daily dosing (s.i.d.) or twice daily dosing (b.i.d.) is maintained for 1 to for example 15 days. Control mice receive the 10% DMSO/0.1% Pluronic® P105 in 0.1% saline without pH adjustment or the 0.25% w/v methylcellulose in water without pH adjustment only.

Three hours after the last dose is administered, the animals are sacrificed by decapitation and trunk blood is collected into 0.5 mL serum separator tubes containing 3.6 mg of a 1:1 weight/weight sodium fluoride: potassium oxalate mixture. The freshly collected samples are centrifuged for two minutes at 10,000 x g at room temperature, and the serum supernatant is transferred and diluted 1:1 volume/volume with a 1TIU/mL aprotinin solution in 0.1% saline without pH adjustment.

The diluted serum samples are then stored at -80°C until analysis. The thawed, diluted serum samples are analyzed for insulin, triglycerides, free fatty acids and cholesterol levels. Serum insulin concentration is determined using Equate® RIA INSULIN kits (double antibody method; as specified by the manufacturer) purchased from Binax, South Portland, ME. The inter assay coefficient of variation is ≤ 10%. Serum triglycerides are determined using the Abbott VP™ and VP Super System® Autoanalyzer (Abbott Laboratories, Irving, TX), or the Abbott Spectrum CCX™ (Abbott Laboratories, Irving, TX) using the A-Gent™ Triglycerides Test reagent system (Abbott Laboratories, Diagnostics Division, Irving, TX) (lipase-coupled enzyme method; a modification of the method of Sampson, et al., Clinical Chemistry 21, 1983 (1975)). Serum total cholesterol levels are determined using the Abbott VP™ and VP Super System® Autoanalyzer (Abbott Laboratories, Irving, TX), or the Abbott Spectrum CCX™ (Abbott Laboratories, Irving, TX) and A-Gent™ Cholesterol Test reagent system (cholesterol esterase-coupled enzyme method; a modification of the method of Allain, et al. Clinical Chemistry 20, 470 (1974)) using a 100 and 300 mg/dL standards. Serum free fatty acid concentration was determined utilizing a kit from Amano International Enzyme Co., Inc., as adapted for use with the Abbott VP™ and VP Super System® Autoanalyzer (Abbott Laboratories, Irving, TX), or the Abbott Spectrum CCX™

(Abbott Laboratories, Irving, TX). Serum insulin, triglycerides, free fatty acids and total cholesterol levels are then calculated by the equations,

$$\text{Serum insulin } (\mu U/mL) = \text{Sample value} \times 2$$

$$\text{Serum triglycerides } (mg/dL) = \text{Sample value} \times 2$$

$$\text{Serum total cholesterol } (mg/dL) = \text{Sample value} \times 2$$

$$\text{Serum free fatty acid } (\mu Eq/L) = \text{Sample value} \times 2 \text{ where 2 is the dilution factor.}$$

The animals are dosed with vehicle maintain substantially unchanged, elevated serum insulin (e.g., 275 μU/mL), serum triglycerides (e.g., 235 mg/dl), serum free fatty acid (1500 mEq/mL) and serum total cholesterol (e.g., 190 mg/dL) levels, while animals treated with compounds of this invention generally display reduced serum insulin, triglycerides, free fatty acid and total cholesterol levels. The serum insulin, triglycerides, free fatty acid and total cholesterol lowering activity of the test compounds are determined by statistical analysis (unpaired t-test) of the mean serum insulin, triglycerides, or total cholesterol concentration between the test compound group and the vehicle-treated control group.

Activity in providing protection from ischemia (e.g., damage to heart tissue) for the compounds of this invention can be demonstrated *in vitro* according to procedures set forth in Butwell et al., Am. J. Physiol., 264, H1884-H1889, 1993 and Allard et al., Am. J. Physio., 1994, 267, H66-H74. Experiments are performed using an isovolumic isolated rat heart preparation, essentially as described in the above-referenced article. Normal male Sprague-Dawley rats, male Sprague-Dawley rats treated to possess cardiac hypertrophy by an aortic banding operation, acutely diabetic male BB/W rats, or non-diabetic BB/W age matched control rats are pretreated with heparin (1000 U, i.p.), followed by pentobarbital (65 mg/kg, i.p.). After deep anesthesia is achieved as determined by the absence of a foot reflex, the heart is rapidly excised and placed into iced saline. The heart is retrogradely perfused through the aorta within 2 minutes. Heart rate and ventricular pressure are determined using a latex balloon in the left ventricle with high pressure tubing connected to a pressure transducer. The heart is perfused with a perfusate solution consisting of (mM) NaCl 118, KCl 4.7, $CaCl_2$ 1.2, $MgCl_2$ 1.2, $NaHCO_3$ 25 and glucose 11. The perfusion apparatus is tightly temperaturecontrolled with heated baths used for the perfusate and for the water jacketing around the perfusion tubing to maintain heart temperature at 37°C. Oxygenation of the perfusate is provided by a pediatric hollow fiber oxygenator (Capiax, Terumo Corp., Tokyo, Japan) immediately proximal to the heart. Hearts are exposed to perfusion solution with and without test compound for about 10 minutes or more, followed by 20 minutes of global ischemia and 60 minutes of reperfusion in the absence of the test compound. The heart beats of the control and test compound treated hearts are compared in the period following ischemia. The left ventricular pressure of the control and test compound treated hearts are compared in the period following ischemia. At the end of the experiment, hearts are also perfused and stained to determine the ratio of infarct area relative to the area at risk (%IA/AAR) as described below.

The therapeutic effects of the compounds of this invention in preventing heart tissue damage otherwise resulting from an ischemic insult can also be demonstrated *in vivo* according to procedures set forth in Liu et al., Circulation, Vol. 84, No. 1, (July 1991), as described specifically herein. The *in vivo* assay tests the cardioprotection of a test compound relative to the control group which receives saline vehicle. As background information, it is noted that brief periods of myocardial ischemia followed by coronary artery reperfusion protects the heart from subsequent severe myocardial ischemia (Murry et al., Circulation 74:1124-1136, 1986). Cardioprotection, as indicated by a reduction in infarcted myocardium, can be induced pharmacologically using intravenously administered adenosine receptor agonists in intact, anesthetized rabbits studied as an *in situ* model of myocardial ischemic preconditioning (Liu et al., Circulation 84:350-356, 1991). The *in vivo* assay tests whether a compound can pharmacologically induce cardioprotection, i.e., reduced myocardial infarct size, when parenterally administered to intact, anesthetized rabbits. The effects of the compounds of this invention can be compared to ischemic preconditioning using the A1 adenosine agonist, $N_6$-1-(phenyl-2R-isopropyl) adenosine (PIA) that has been shown to pharmacologically induce cardioprotection in intact anesthetized rabbits studied *in situ* (Liu et al., Circulation 84:350-356, 1991). The exact methodology is described below.

<u>Surgery:</u> New Zealand White male rabbits (3-4 kg) are anesthetized with sodium pentobarbital (30 mg/kg, i.v.). A tracheotomy is performed via a ventral midline cervical incision and the rabbits are ventilated with 100% oxygen using a positive pressure ventilator. Catheters are placed in the left jugular vein for compound administration and in the left carotid artery for blood pressure measurements. The hearts are then exposed through a left thoracotomy and

a snare (00 silk) placed around a prominent branch of the left coronary artery. Ischemia is induced by pulling the snare tight and clamping it in place. Releasing the snare allowed the affected area to reperfuse. Myocardial ischemia is evidenced by regional cyanosis; reperfusion was evidenced by reactive hyperemia.

Protocol: Once arterial pressure and heart rate has been stable for at least 30 minutes the experiment is started. Ischemic preconditioning is induced by twice occluding the coronary artery for 5 min followed by a 10 min reperfusion. Pharmacological preconditioning is induced by twice infusing a test compound over, for example, 5 minutes and allowing 10 minutes before further intervention or by infusing the adenosine agonist, PIA (0.25 mg/kg). Following ischemic preconditioning, pharmacological preconditioning or no conditioning (unconditioned, vehicle control) the artery is occluded for 30 minutes and then reperfused for two hours to induce myocardial infarction. The test compound and PIA are dissolved in saline or other suitable vehicle and delivered at 1 to 5 ml/kg, respectively.

Staining (Liu et al., *Circulation* 84:350-356, 1991): At the end of the 2 hour reperfusion period, the hearts are quickly removed, hung on a Langendorff apparatus, and flushed for 1 minute with normal saline heated to body temperature (38°C). The silk suture used as the snare is then tied tightly to reocclude the artery and a 0.5% suspension of fluorescent particles (1-10 μm in diameter, obtained from Duke Scientific Corp., Palo Alto, CA) is infused with the perfusate to stain all of the myocardium except the area at risk (nonfluorescent ventricle). The hearts are then quickly frozen and stored overnight at -20°C. On the following day, the hearts are cut into 2 mm slices and stained with 1% triphenyl tetrazolium chloride (TTC). Since TTC reacts with living tissue, this stain differentiates between living (red stained) tissue, and dead tissue (unstained infarcted tissue). The infarcted area (no stain) and the area at risk (no fluorescent particles) are calculated for each slice of left ventricle using a pre-calibrated image analyzer. To normalize the ischemic injury for differences in the area at risk between hearts, the data is expressed as the ratio of infarct area vs. area at risk (%IA/AAR). All data are expressed as Mean±SEM and compared statistically using single factor ANOVA or unpaired t-test. Significance is considered as $p<0.05$.

The invention can be tested for its utility in reducing or preventing ischemic injury in non-cardiac tissues, for example, the brain, or the liver, utilizing procedures reported in the scientific literature. The invention can be administered by the preferred route and vehicle of administration and at the preferred time of administration either prior to the ischemic episode, during the ischemic episode, following the ischemic episode (reperfusion period) if included, or during any of the below-mentioned experimental stages.

The benefit of the invention to reduce ischemic brain damage can be demonstrated, for example, in mammals using the method of Park, et al (Ann. Neurol. 1988;24:543-551). In brief, in the procedure of Park, et al. adult male sprague Dawley rats are anesthetized initially with 2% halothane, and thereafter by mechanical ventilation with a nitrous oxide-oxygen mixture (70%:30%) containing 0.5-1% halothane. A tracheostomy is then performed. The stroke volume of the ventilator is adjusted to maintain arterial carbon dioxide tension at approximately 35 mm Hg and adequate arterial oxygenation ($PaO_2$>90 mm Hg). Body temperature can be monitored by a rectal thermometer, and the animals can be maintained normothermic, if necessary, by external heating. The animals next undergo subtemporal craniectomy to expose the main trunk of the left middle cerebral artery (MCA) under an operating microscope, and the exposed artery is occluded with microbipolar coagulation to generate large ischemic lesions in the cerebral cortex and basal ganglia. After three hours of MCA occlusion, the rats are deeply anesthetized with 2% halothane and a thoracotomy is performed to infuse heparinized saline into the left ventricle. The effluent is collected via an incision of the right atrium. The saline washout is followed by approximately 200 ml of a 40% formaldehyde, glacial acetic acid and absolute methanol solution (FAM; 1:1:8, v/v/v), then the animals are decapitated and the head is stored in the fixative for 24 hours. The brain is then removed, dissected, processed, embedded in paraffin wax, and sectioned (approximately 100 sections per brain). The sections are then stained with hematoxylin-eosin or with a combination of cresyl violet and Luxol fast blue, and examined by light microscopy to identify and quantitate the ischemic damage using an image analyzer (e.g. the Quantimet 720). The ischemic volumes and areas are expressed in absolute units ($mm^3$ and $mm^2$) and as a percentage of the total region examined. The effect of the invention to reduce ischemic brain damage induced by MCA occlusion is noted based on a reduction in the area or volume of relative or absolute ischemic damage in the brain sections from the rats in the treatment group compared to brain sections from rats in a placebo-treated control group.

Other methods which could alternatively be utilized to demonstrate the benefit of the invention to reduce ischemic brain damage include those described by Nakayama, et al. in Neurology 1988;38:1667-1673, Memezawa, et al. in Stroke 1992;23:552-559, Folbergrova, et al. in Proc. Natl. Acad. Sci 1995;92:5057-5059, or Gotti, et al. in Brain Res. 1990;522:290-307.

The benefit of the invention to reduce ischemic liver damage can be demonstrated, for example, in mammals using the method of Yokoyama, et al. (Am. J. Physiol. 1990;258:G564-G570). In brief, in the procedure of Yokoyama, et al., fasted adult male Sprague Dawley rats are anesthetized with sodium pentobarbital (40 mg/kg i.p.), then the animals are tracheotomized and mechanically ventilated with room air. The liver is extirpated and placed in an environmental chamber maintained at constant temperature (37 °C), then perfused through the portal vein at a constant pressure of 15 cm $H_2O$ with a modified, hemoglobin-free Krebs-Henseleit buffer (in mM: 118 NaCl, 4.7 KCl, 27 $NaHCO_3$,

2.5 $CaCl_2$, 1.2 $MgSO_4$, 1.2 $KH_2PO_4$, 0.05 EDTA, and 11 mM glucose, plus 300 U heparin). The pH of the perfusate is maintained at 7.4 by gassing the buffer with 95% $O_2$ - 5% $CO_2$. Each liver is perfused at a flow rate of 20 ml/min in a single-pass manner for a 30 min washout and equilibration period (preischemic period), followed by a 2 hour period of global ischemia, and then a 2 hour period of reperfusion under conditions identical to the preischemic period. Aliquots (20 ml) of the perfusate are collected during the preischemic period, immediately after the occlusive ischemic period, and every 30 min of the 2 hour reperfusion period. The perfusate samples are assayed for the appearance of hepatocellular enzymes, for example, aspartate amino-transferase (AST), alanine amino-transferase (ALT), and lactate dehydrogenase (LDH), which are taken to quantitatively reflect the degree of ischemic liver tissue damage during the procedure. AST, ALT, and LDH activities in the perfusate can be determined by several methods, for example, by the reflectometry method using an automatic Kodak Ektachem 500 analyzer reported by Nakano, et al. (Hepatology 1995; 22:539-545). The effect of the invention to reduce ischemic liver damage induced by occlusion is noted based on a reduction in the release of hepatocellular enzymes immediately following the occlusive period and/or during the postischemic-reperfusion period in the perfused livers from the rats in the treatment group compared to perfused livers from rats in a placebo-treated control group.

Other methods and parameters which could alternatively be utilized to demonstrate the benefit of the invention to reduce ischemic liver damage include those described by Nakano, et al. (Hepatology 1995;22:539-545).

It should be understood that the invention is not limited to the particular embodiments described herein, but that various changes and modifications may be made without departing from the spirit and scope of this novel concept as defined bythe following claims.

## Claims

1. A binding site in a glycogen phosphorylase enzyme for a glycogen phosphorylase inhibitor in which a portion of said inhibitor is in van der Waals or hydrogen bonding contact with a portion or all portions of the following residues of said glycogen phosphorylase enzyme:

| parent secondary structure | residue number |
|---|---|
| | 13-23 |
| helix α1 | 24-37 |
| turn | 38-39, 43, 46-47 |
| helix α2 | 48-66, 69-70, 73-74, 76-78 |
| | 79-80 |
| strand β1 | 81-86 |
| | 87-88 |
| strand β2 | 89-92 |
| | 93 |
| helix α3 | 94-102 |
| | 103 |
| helix α4 | 104-115 |
| | 116-117 |
| helix α5 | 118-124 |
| | 125-128 |
| strand β3 | 129-131 |
| | 132-133 |
| helix α6 | 134-150 |
| | 151-152 |
| strand β4 | 153-160 |
| | 161 |
| strand β4b | 162-163 |
| | 164-166 |
| strand β5 | 167-171 |
| | 172-173 |
| strand β6 | 174-178 |

(continued)

| parent secondary structure | residue number |
|---|---|
| | 179-190 |
| strand β7 | 191-192 |
| | 194,197 |
| strand β8 | 198-209 |
| | 210-211 |
| strand β9 | 212-216 |
| strand β10 | 219-226, 228-232 |
| | 233-236 |
| strand β11 | 237-239, 241, 243-247 |
| | 248-260 |
| helix α7 | 261-276 |
| strand β11b | 277-281 |
| reverse turn | 282-289 |
| helix α8 | 290-304 |

**2.** A binding site according to claim 1 in which a portion of said inhibitor is in van der Waals or hydrogen bonding contact with a portion of all portions of the following residues of glycogen phosphorylase in one or both subunits:

| parent secondary structure | residue number |
|---|---|
| | 13-23 |
| helix α1 | 24-37 |
| turn | 38-39, 43, 46-47 |
| helix α2 | 48-66, 69-70, 73-74, 76-78 |
| | 79-80 |
| strand β2 | 91-92 |
| | 93 |
| helix α3 | 94-102 |
| | 103 |
| helix α4 | 104-115 |
| | 116-117 |
| helix α5 | 118-124 |
| | 125-128 |
| strand β3 | 129-130 |
| strand β4 | 159-160 |
| | 161 |
| strand β4b | 162-163 |
| | 164-166 |
| strand β5 | 167-168 |
| strand β6 | 178 |
| | 179-190 |
| strand β7 | 191-192 |
| | 194,197 |
| strand β9 | 198-200 |
| strand β10 | 220-226,<br>228-232 |
| | 233-236 |
| strand β11 | 237-239, 241, 243-247 |
| | 248-260 |
| helix α7 | 261-276 |

(continued)

| parent secondary structure | residue number |
|---|---|
| strand β11b | 277-280 |

**3.** A binding site for a glycogen phosphorylase inhibitor according to claim 2, in which a portion of said inhibitor is in van der Waals or hydrogen bonding contact with a portion or all portions of the following residues of glycogen phosphorylase in one or both subunits:

| residue number |
|---|
| 33-39 |
| 49-66 |
| 94 |
| 98 |
| 102 |
| 125-126 |
| 160 |
| 162 |
| 182-192 |
| 197 |
| 224-226 |
| 228-231 |
| 238-239 |
| 241 |
| 245 |
| 247 |

**4.** A binding site for a glycogen phosphorylase inhibitor according to claim 3, in which a portion of said inhibitor is in van der Waals or hydrogen bonding contact with a portion or all portions of the following residues of glycogen phosphorylase in one or both subunits:

| residue number |
|---|
| 37-39 |
| 53 |
| 57 |
| 60 |
| 63-64 |
| 184-192 |
| 226 |
| 229 |

**5.** A complex of glycogen phosphorylase with a glycogen phosphorylase inhibitor in which a portion of said inhibitor is in van der Waals or hydrogen bonding contact with a portion or all portions of the following residues of glycogen phosphorylase in one or both subunits:

| residue number |
|---|
| 37-39 |
| 53 |
| 57 |
| 60 |
| 63-64 |
| 184-192 |
| 226 |
| 229 |

**6.** A crystal of a complex of Claim 5.

**7.** A crystal of Claim 6,
wherein the glycogen phosphorylase inhibitor is of Formula I

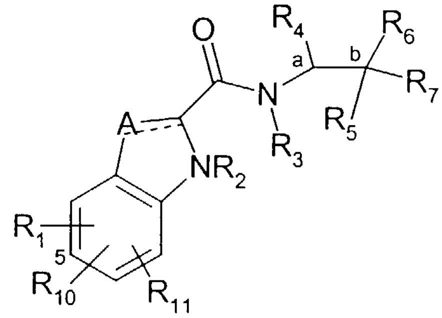

Formula I

or the pharmaceutically acceptable salts and prodrugs thereof
wherein
the dotted line (---) is an optional bond;

A is -C(H)=, -C(($C_1$-$C_4$)alkyl)= or -C(halo)= when the dotted line (---) is a bond, or A is methylene or -CH(($C_1$-$C_4$)alkyl)- when the dotted line (---) is not a bond;
$R_1$, $R_{10}$ or $R_{11}$ are each independently H, halo, 4-, 6- or 7-nitro, cyano, ($C_1$-$C_4$)alkyl, ($C_1$-$C_4$)alkoxy, fluoromethyl, difluoromethyl or trifluoromethyl;
$R_2$ is H;
$R_3$ is H or ($C_1$-$C_5$)alkyl;
$R_4$ is methyl, ethyl, n-propyl, hydroxy($C_1$-$C_3$)alkyl, ($C_1$-$C_3$)alkoxy($C_1$-$C_3$)alkyl, phenyl($C_1$-$C_4$)alkyl, phenylhydroxy($C_1$-$C_4$)alkyl, phenyl($C_1$-$C_4$)alkoxy($C_1$-$C_4$)alkyl, thien-2- or -3-yl($C_1$-$C_4$)alkyl or fur-2- or -3-yl($C_1$-$C_4$)alkyl wherein said $R_4$ rings are mono-, di- or tri-substituted independently on carbon with H, halo, ($C_1$-$C_4$)alkyl, ($C_1$-$C_4$)alkoxy, trifluoromethyl, hydroxy, amino or cyano; or
$R_4$ is pyrid-2-, -3- or -4-yl($C_1$-$C_4$)alkyl, thiazol-2-, -4- or -5-yl($C_1$-$C_4$)alkyl, imidazol -1-,-2-, -4- or -5-yl($C_1$-$C_4$) alkyl, pyrrol-2- or -3-yl($C_1$-$C_4$)alkyl, oxazol-2-, -4- or -5-yl-($C_1$-$C_4$)alkyl, pyrazol-3-, -4- or -5-yl($C_1$-$C_4$)alkyl, isoxazol-3-, -4- or -5-yl($C_1$-$C_4$)alkyl, isothiazol-3-, -4- or -5-yl($C_1$-$C_4$)alkyl, pyridazin-3- or -4-yl-($C_1$-$C_4$)alkyl, pyrimidin-2-, -4-, -5- or -6-yl($C_1$-$C_4$)alkyl, pyrazin-2- or -3-yl($C_1$-$C_4$)alkyl or 1,3,5-triazin-2-yl($C_1$-$C_4$)alkyl, wherein said preceding $R_4$ heterocycles are optionally mono- or di-substituted independently with halo, trifluoromethyl, ($C_1$-$C_4$)alkyl, ($C_1$-$C_4$)alkoxy, amino or hydroxy and said mono-or di-substituents are bonded to carbon;
$R_5$ is hydroxy, fluoro, ($C_1$-$C_5$)alkyl, ($C_1$-$C_5$)alkoxy, ($C_1$-$C_6$)alkanoyl, amino($C_1$-$C_4$)alkoxy, mono-N- or di-N,N-($C_1$-$C_4$)alkylamino($C_1$-$C_4$)alkoxy, carboxy($C_1$-$C_4$)alkoxy, ($C_1$-$C_5$)alkoxycarbonyl($C_1$-$C_4$)alkoxy, benzyloxycarbonyl($C_1$-$C_4$)alkoxy, or carbonyloxy wherein said carbonyloxy is carbon-carbon linked with phenyl, thiazolyl, imidazolyl, 1H-indolyl, furyl, pyrrolyl, oxazolyl, pyrazolyl, isoxazolyl, isothiazolyl, pyridazinyl, pyrimidinyl, pyrazinyl or 1,3,5-triazinyl and wherein said preceding $R_5$ rings are optionally mono-substituted with halo, ($C_1$-$C_4$)alkyl, ($C_1$-$C_4$)alkoxy, hydroxy, amino or trifluoromethyl and said mono-substituents are bonded to carbon;
$R_7$ is H, fluoro or ($C_1$-$C_5$)alkyl; or
$R_5$ and $R_7$ can be taken together to be oxo;
$R_6$ is carboxy or ($C_1$-$C_8$)alkoxycarbonyl, C(O)N$R_8R_9$ or C(O)$R_{12}$,

wherein

$R_8$ is H, ($C_1$-$C_3$)alkyl, hydroxy or ($C_1$-$C_3$)alkoxy; and
$R_9$ is H, ($C_1$-$C_8$)alkyl, hydroxy, ($C_1$-$C_8$)alkoxy, methylene-perfluorinated($C_1$-$C_8$)alkyl, phenyl, pyridyl, thienyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, pyridazinyl, pyrimidinyl, pyrazinyl, piperazinyl or 1,3,5-triazinyl wherein said preceding $R_9$ rings are carbon-nitrogen linked; or
$R_9$ is mono-, di- or tri-substituted ($C_1$-$C_5$)alkyl, wherein said substituents are independently H, hydroxy, amino,

mono-N- or di-N,N-($C_1$-$C_5$)alkylamino; or
$R_9$ is mono- or di-substituted ($C_1$-$C_5$)alkyl, wherein said substituents are independently phenyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl or 1,3,5-triazinyl wherein the nonaromatic nitrogen-containing $R_9$ rings are optionally mono-substituted on nitrogen with ($C_1$-$C_6$)alkyl, benzyl, benzoyl or ($C_1$-$C_6$)alkoxycarbonyl and wherein the $R_9$ rings are optionally mono-substituted on carbon with halo, ($C_1$-$C_4$)alkyl, ($C_1$-$C_4$)alkoxy, hydroxy, amino, or mono-N- and di-N,N ($C_1$-$C_5$)alkylamino provided that no quaternized nitrogen is included and there are no nitrogen-oxygen, nitrogen-nitrogen or nitrogen-halo bonds;
$R_{12}$ is piperazin-1-yl, 4-($C_1$-$C_4$)alkylpiperazin-1-yl, 4-formylpiperazin-1-yl, morpholino, thiomorpholino, 1-oxothiomorpholino, 1,1-dioxo-thiomorpholino, thiazolidin-3-yl, 1-oxothiazolidin-3-yl, 1,1-dioxo-thiazolidin-3-yl, 2-($C_1$-$C_6$)alkoxycarbonylpyrrolidin-1-yl, oxazolidin-3-yl or 2(R)-hydroxymethylpyrrolidin-1-yl; or
$R_{12}$ is 3- and/or 4-mono-or di-substituted oxazetidin-2-yl, 2-, 4-, and/or 5- mono- or di-substituted oxazolidin-3-yl, 2-, 4-, and/or 5- mono- or di- substituted thiazolidin-3-yl, 2-, 4-, and/or 5- mono- or di- substituted 1-oxothiazolidin-3-yl, 2-, 4-, and/or 5- mono- or di-substituted 1,1-dioxothiazolidin-3-yl, 3- and/or 4-, mono- or di-substituted pyrrolidin-1-yl, 3-, 4-and/or 5-, mono-, di- or tri-substituted piperidin-1-yl, 3-, 4-, and/or 5- mono-, di-, or tri-substituted piperazin-1-yl, 3-substituted azetidin-1-yl, 4- and/or 5-, mono- or di-substituted 1,2-oxazinan-2-yl, 3-and/or 4-mono- or di-substituted pyrazolidin-1-yl, 4- and/or 5-, mono- or di-substituted isoxazolidin-2-yl, 4- and/or 5-, mono- and/or di-substituted isothiazolidin-2-yl wherein said $R_{12}$ substituents are independently H, halo, ($C_1$-$C_5$)-alkyl, hydroxy, amino, mono-N- or di-N,N-($C_1$-$C_5$)alkylamino, formyl, oxo, hydroxyimino, ($C_1$-$C_5$)alkoxy, carboxy, carbamoyl, mono-N-or di-N,N-($C_1$-$C_4$)alkylcarbamoyl, ($C_1$-$C_4$)alkoxyimino, ($C_1$-$C_4$)alkoxymethoxy, ($C_1$-$C_6$)alkoxycarbonyl, carboxy($C_1$-$C_5$)alkyl or hydroxy($C_1$-$C_5$)alkyl;
with the proviso that if $R_4$ is H, methyl, ethyl or n-propyl, $R_5$ is OH;
with the proviso that if $R_5$ and $R_7$ are H, then $R_4$ is not H, methyl, ethyl, n-propyl, hydroxy($C_1$-$C_3$)alkyl or ($C_1$-$C_3$)alkoxy($C_1$-$C_3$)alkyl and $R_6$ is C(O)$NR_8R_9$, C(O)$R_{12}$ or ($C_1$-$C_4$)alkoxycarbonyl.

**8.** A crystal of Claim 6, wherein
the glycogen phosphorylase inhibitor is of Formula II

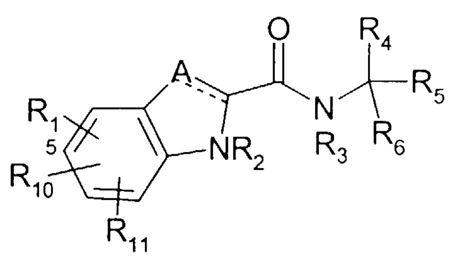

Formula II

or the pharmaceutically acceptable salts and prodrugs thereof
wherein
the dotted line (---) is an optional bond;

A is -C(H)=, -C(($C_1$-$C_4$)alkyl)=, -C(halo)= or -N=, when the dotted line (---) is a bond, or A is methylene or -CH(($C_1$-$C_4$)alkyl)-, when the dotted line (---) is not a bond;
$R_1$, $R_{10}$ or $R_{11}$ are each independently H, halo, cyano, 4-, 6-, or 7-nitro, ($C_1$-$C_4$)alkyl, ($C_1$-$C_4$)alkoxy, fluoromethyl, difluoromethyl or trifluoromethyl;
$R_2$ is H;
$R_3$ is H or ($C_1$-$C_5$)alkyl;
$R_4$ is H, methyl, ethyl, n-propyl, hydroxy($C_1$-$C_3$)alkyl, ($C_1$-$C_3$)alkoxy($C_1$-$C_3$)alkyl, phenyl($C_1$-$C_4$)alkyl, phenylhydroxy($C_1$-$C_4$)alkyl, (phenyl)(($C_1$-$C_4$)-alkoxy)($C_1$-$C_4$)alkyl, thien-2- or -3-yl($C_1$-$C_4$)alkyl or fur-2- or -3-yl($C_1$-$C_4$)alkyl wherein said $R_4$ rings are mono-, di- or tri-substituted independently on carbon with H, halo, ($C_1$-$C_4$) alkyl, ($C_1$-$C_4$)alkoxy, trifluoromethyl, hydroxy, amino, cyano or 4,5-dihydro-1H-imidazol-2-yl; or
$R_4$ is pyrid-2-, -3- or -4-yl($C_1$-$C_4$)alkyl, thiazol-2-, -4- or -5-yl($C_1$-$C_4$)alkyl, imidazol-2-,-4- or -5-yl($C_1$-$C_4$)alkyl,

pyrrol-2- or -3-yl($C_1$-$C_4$)alkyl, oxazol-2-, -4- or -5-yl($C_1$-$C_4$)alkyl, pyrazol-3-, -4- or -5-yl($C_1$-$C_4$)alkyl, isoxazol-3-, -4- or -5-yl($C_1$-$C_4$)alkyl, isothiazol-3-, -4- or -5-yl($C_1$-$C_4$)alkyl, pyridazin-3- or -4-yl($C_1$-$C_4$)alkyl, pyrimidin-2-, -4-, -5- or -6-yl($C_1$-$C_4$)alkyl, pyrazin-2- or -3-yl($C_1$-$C_4$)alkyl, 1,3,5-triazin-2-yl($C_1$-$C_4$)alkyl or indol-2-($C_1$-$C_4$) alkyl, wherein said preceding $R_4$ heterocycles are optionally mono- or di-substituted independently with halo, trifluoromethyl, ($C_1$-$C_4$)alkyl, ($C_1$-$C_4$)alkoxy, amino, hydroxy or cyano and said substituents are bonded to carbon; or

$R_4$ is $R_{15}$-carbonyloxymethyl, wherein said $R_{15}$ is phenyl, thiazolyl, imidazolyl, 1H-indolyl, furyl, pyrrolyl, oxazolyl, pyrazolyl, isoxazolyl, isothiazolyl, pyridyl, pyridazinyl, pyrimidinyl, pyrazinyl or 1,3,5-triazinyl and wherein said preceding $R_{15}$ rings are optionally mono- or di-substituted independently with halo, amino, hydroxy, ($C_1$-$C_4$)alkyl, ($C_1$-$C_4$)alkoxy or trifluoromethyl and said mono- or di-substituents are bonded to carbon;

$R_5$ is H, methyl, ethyl, n-propyl, hydroxymethyl or hydroxyethyl;

$R_6$ is carboxy, ($C_1$-$C_8$)alkoxycarbonyl, or benzyloxycarbonyl,

wherein

$R_8$ is ($C_1$-$C_6$)alkyl, cyclo($C_3$-$C_6$)alkyl, cyclo($C_3$-$C_6$)alkyl($C_1$-$C_5$)alkyl, hydroxy or ($C_1$-$C_8$)alkoxy; and

$R_9$ is cyclo($C_3$-$C_8$)alkyl, cyclo($C_3$-$C_8$)alkyl($C_1$-$C_5$)alkyl, cyclo($C_4$-$C_7$)alkenyl, cyclo($C_3$-$C_7$)alkyl($C_1$-$C_5$)alkoxy, cyclo($C_3$-$C_7$)alkyloxy, hydroxy, methylene-perfluorinated($C_1$-$C_8$)alkyl, phenyl, or a heterocycle wherein said heterocycle is pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl, 1,3,5-triazinyl, benzothiazolyl, benzoxazolyl, benzimidazolyl, thiochromanyl or tetrahydrobenzothiazolyl wherein said heterocycle rings are carbon-nitrogen linked; or

$R_9$ is ($C_1$-$C_6$)alkyl or ($C_1$-$C_8$)alkoxy wherein said ($C_1$-$C_6$)alkyl or ($C_1$-$C_8$)alkoxy is optionally monosubstituted with cyclo($C_4$-$C_7$)alken-1-yl, phenyl, thienyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, thiomorpholinyl, 1-oxothiomorpholinyl, 1,1-dioxothiomorpholinyl, pyridazinyl, pyrimidinyl, pyrazinyl, piperazinyl, 1,3,5-triazinyl or indolyl and wherein said ($C_1$-$C_6$)alkyl or ($C_1$-$C_8$)alkoxy are optionally additionally independently mono-or di-substituted with halo, hydroxy, ($C_1$-$C_5$)alkoxy, amino, mono-N- or di-N,N-($C_1$-$C_5$)alkylamino, cyano, carboxy, or ($C_1$-$C_4$)alkoxycarbonyl; and

wherein the $R_9$ rings are optionally mono- or di-substituted independently on carbon with halo, ($C_1$-$C_4$)alkyl, ($C_1$-$C_4$)alkoxy, hydroxy, hydroxy($C_1$-$C_4$)alkyl, amino($C_1$-$C_4$)alkyl, mono-N- or di-N,N-($C_1$-$C_4$)alkylamino($C_1$-$C_4$)alkyl, ($C_1$-$C_4$)alkoxy($C_1$-$C_4$)alkyl, amino, mono-N- or di-N,N-($C_1$-$C_4$)alkylamino, cyano, carboxy, ($C_1$-$C_5$) alkoxycarbonyl, carbamoyl, formyl or trifluoromethyl and said $R_9$ rings may optionally be additionally mono- or di-substituted independently with ($C_1$-$C_5$)alkyl or halo;

with the proviso that no quaternized nitrogen on any $R_9$ heterocycle is included;

$R_{12}$ is morpholino, thiomorpholino, 1-oxothiomorpholino, 1,1-dioxothiomorpholino, thiazolidin-3-yl, 1-oxothiazolidin-3-yl, 1,1-dioxothiazolidin-3-yl, pyrrolidin-1-yl, piperidin-1-yl, piperazin-1-yl, piperazin-4-yl, azetidin-1-yl, 1,2-oxazinan-2-yl, pyrazolidin-1-yl, isoxazolidin-2-yl, isothiazolidin-2-yl, 1,2-oxazetidin-2-yl, oxazolidin-3-yl, 3,4-dihydroisoquinolin-2-yl, 1,3-dihydroisoindol-2-yl, 3,4-dihydro-2H-quinol-1-yl, 2,3-dihydro-benzo[1,4]oxazin-4-yl, 2,3-dihydro-benzo[1,4]-thiazine-4-yl, 3,4-dihydro-2H-quinoxalin-1-yl, 3,4-dihydro-benzo[c][1,2]oxazin-1-yl, 1,4-dihydro-benzo[d][1,2]oxazin-3-yl, 3,4-dihydro-benzo[e][1,2]-oxazin-2-yl, 3H-benzo[d]isoxazol-2-yl, 3H-benzo[c]isoxazol-1 -yl or azepan-1-yl,

wherein said $R_{12}$ rings are optionally mono-, di- or tri-substituted independently with halo, ($C_1$-$C_5$)alkyl, ($C_1$-$C_5$)alkoxy, hydroxy, amino, mono-N- or di-N,N-($C_1$-$C_5$)alkylamino, formyl, carboxy, carbamoyl, mono-N- or di-N,N-($C_1$-$C_5$)alkylcarbamoyl, ($C_1$-$C_6$)alkoxy($C_1$-$C_3$)alkoxy, ($C_1$-$C_5$)alkoxycarbonyl, benzyloxycarbonyl, ($C_1$-$C_5$) alkoxycarbonyl($C_1$-$C_5$)alkyl, ($C_1$-$C_4$)alkoxycarbonylamino, carboxy($C_1$-$C_5$)alkyl, carbamoyl($C_1$-$C_5$)alkyl, mono-N- or di-N,N-($C_1$-$C_5$)alkylcarbamoyl($C_1$-$C_5$)alkyl, hydroxy($C_1$-$C_5$)alkyl, ($C_1$-$C_4$)alkoxy($C_1$-$C_4$)alkyl, amino ($C_1$-$C_4$)alkyl, mono-N- or di-N,N-($C_1$-$C_4$)alkylamino($C_1$-$C_4$)alkyl, oxo, hydroxyimino or ($C_1$-$C_6$)alkoxyimino and wherein no more than two substituents are selected from oxo, hydroxyimino or ($C_1$-$C_6$)alkoxyimino and oxo, hydroxyimino or ($C_1$-$C_6$)alkoxyimino are on nonaromatic carbon; and

wherein said $R_{12}$ rings are optionally additionally mono- or di-substituted independently with ($C_1$-$C_5$)alkyl or halo;

with the proviso that when $R_6$ is ($C_1$-$C_5$)alkoxycarbonyl or benzyloxycarbonyl then $R_1$ is 5-halo, 5-($C_1$-$C_4$)alkyl or 5-cyano and $R_4$ is (phenyl)(hydroxy)($C_1$-$C_4$)alkyl, (phenyl)(($C_1$-$C_4$)alkoxy)($C_1$-$C_4$)alkyl, hydroxymethyl or Ar ($C_1$-$C_2$)alkyl, wherein Ar is thien-2- or -3-yl, fur-2-or -3-yl or phenyl wherein said Ar is optionally mono- or di-substituted independently with halo; with the provisos that when $R_4$ is benzyl and $R_5$ is methyl, $R_{12}$ is not 4-hydroxy-piperidin-1-yl or when $R_4$ is benzyl and $R_5$ is methyl $R_6$ is not $C(O)N(CH_3)_2$;

with the proviso that when $R_1$ and $R_{10}$ and $R_{11}$ are H, $R_4$ is not imidazol-4-ylmethyl, 2-phenylethyl or 2-hydroxy-2-phenylethyl;
with the proviso that when both $R_8$ and $R_9$ are n-pentyl, $R_1$ is 5-chloro, 5-bromo, 5-cyano, 5($C_1$-$C_5$)alkyl, 5($C_1$-$C_5$)alkoxy or trifluoromethyl;
with the proviso that when $R_{12}$ is 3,4-dihydroisoquinol-2-yl, said 3,4-dihydroisoquinol-2-yl is not substituted with carboxy(($C_1$-$C_4$)alkyl;
with the proviso that when $R_8$ is H and $R_9$ is ($C_1$-$C_6$)alkyl, $R_9$ is not substituted with carboxy or ($C_1$-$C_4$)alkoxycarbonyl on the carbon which is attached to the nitrogen atom N of $NHR_9$; and
with the proviso that when $R_6$ is carboxy and $R_1$, $R_{10}$, $R_{11}$ and $R_5$ are all H, then $R_4$ is not benzyl, H, (phenyl)(hydroxy)methyl, methyl, ethyl or n-propyl.

**9.** A crystal of Claim 6,
wherein the glycogen phosphorylase inhibitor is of formula III

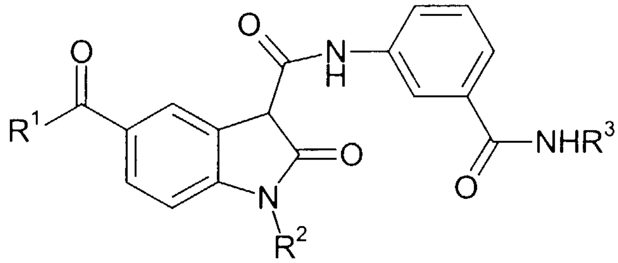

III

or a prodrug thereof or a pharmaceutically acceptable salt of said compound or said prodrug wherein:

$R^1$ is ($C_1$-$C_4$)alkyl, ($C_3$-$C_7$)cycloalkyl, phenyl or phenyl substituted with up to three ($C_1$-$C_4$)alkyl, ($C_1$-$C_4$)alkoxy or halogen;
$R^2$ is ($C_1$-$C_4$)alkyl; and
$R^3$ is ($C_3$-$C_7$)cycloalkyl; phenyl; phenyl substituted at the para position with ($C_1$-$C_4$)alkyl, halo, hydroxy($C_1$-$C_4$)alkyl or trifluoromethyl; phenyl substituted at the meta position with fluoro; or phenyl substituted at the ortho position with fluoro.

**10.** A crystal of Claim 7 wherein the glycogen phosphorylase is human liver glycogen phosphorylase and has the following physical measurements:

| | |
|---|---|
| Space group | $P3_1$ |
| Unit Cell: | a = b = 124.63 angstroms<br>c = 124.06 angstroms<br>$\alpha = \beta$ = 90.00 degrees<br>$\delta$ = 120.00 degrees |

said values measured in a unit cell having the following variability of the dimensions.

| | | |
|---|---|---|
| a | 123.4 - | 124 63 Å (1%) |
| c | 122.22 - | 124.06 Å (1.5%) |

**11.** A crystal of Claim 8 wherein the glycogen phosphorylase is human liver glycogen phosphorylase and has the following physical measurements:

| | |
|---|---|
| Space group | $P3_1$ |
| Unit Cell: | a = b = 124.63 angstroms |

(continued)

| | |
|---|---|
| | c = 124.06 angstroms<br>$\alpha = \beta$ = 90.00 degrees<br>$\delta$ = 120.00 degrees |

said values measured in a unit cell having the following variability of the dimensions.

| | | |
|---|---|---|
| a | 123.4 - | 12463 Å (1%) |
| c | 122.22 - | 124.06 Å (1.5%) |

**12.** A crystal of Claim 9 wherein the glycogen phosphorylase is human liver glycogen phosphorylase and has the following physical measurements:

| | |
|---|---|
| Space group<br>Unit Cell: | $P3_1$<br>a = b = 124.63 angstroms<br>c = 124.06 angstroms<br>$\alpha = \beta$ = 90.00 degrees<br>$\delta$ = 120.00 degrees |

said values measured in a unit cell having the following variability of the dimensions.

| | | |
|---|---|---|
| a | 123.4 - | 124 63 Å (1%) |
| c | 122.22 - | 124.06 Å (1.5%) |

**13.** A method of detecting the formation of a complex of Claim 5.

**14.** A method of Claim 13 which relies on displacement of a bound ligand from the binding site.

**15.** A method of determining inhibition of human liver glycogen phosphorylase which is based on formation of a complex of Claim 5.

**16.** A method for designing an inhibitor of human liver glycogen phosphorylase, said method comprising steps a-c below:

a) forming a complex of a compound binding to glycogen phosphorylase
b) determining structural features of the complex in step (a), in particular those of the binding site for said compound, and
c) using the structural features determined in step (b), in particular those of the binding site for said compound of step (a), or a model based on said structural features, to design a human liver glycogen phosphorylase inhibitor capable of binding to the binding site of Claim 2.

**17.** A method of treatment of a mammal having an indication due to hyperglycemia, hyperinsulimemia, hyperlipidemia, insulin resistance or tissue ischemia comprising:

a) forming a complex of a compound binding to glycogen phosphorylase
b) determining structural features of the complex in step (a), in particular those of the binding site for said compound and
c) using the structural features determined in step (b), in particular those of the binding site for said compound of step (a), or a model based on said structural features, to design a human liver glycogen phosphorylase inhibitor capable of binding to the binding site of Claim 2.
d) administering the compound of step c) to a patient in need of such treatment.

European Patent Office

# EUROPEAN SEARCH REPORT

Application Number
EP 99 30 6047

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D<br><br><br>Y | WO 96 39385 A (TREADWAY JUDITH L ;HULIN BERNARD (US); HOOVER DENNIS J (US); PFIZER) 12 December 1996 (1996-12-12)<br>* page 5, line 10 - page 8, line 3 *<br>* page 47, line 3 - page 48, line 27 *<br>* page 49, line 25 - page 51, line 7 *<br>* the examples *<br>--- | 1-5,<br>13-15<br><br>6-12 | A61K31/404<br>C07D209/42<br>C12Q1/48<br>A61P5/50<br>A61P9/10 |
| X,D<br><br><br>Y | WO 96 39384 A (TREADWAY JUDITH L ;HULIN BERNARD (US); HOOVER DENNIS J (US); PFIZER) 12 December 1996 (1996-12-12)<br>* page 5, line 10 - page 10, line 4 *<br>* page 42, line 27 - page 44, line 18 *<br>* page 45, line 17 - page 46, line 32 *<br>* the examples *<br>--- | 1-5,<br>13-15<br><br>6-12 | |
| X<br><br>Y | EP 0 846 464 A (PFIZER)<br>10 June 1998 (1998-06-10)<br>* page 2, line 33 - page 3, line 56 *<br>* page 8, line 18 - page 9, line 48 *<br>--- | 1-5,<br>13-15<br>6-12 | |
| X<br><br><br><br><br>Y | HOOVER, DENNIS J. ET AL: "Indole-2-carboxamide inhibitors of human liver glycogen phosphorylase"<br>J. MED. CHEM. (1998), 41(16), 2934-2938 , XP002122958<br>* the whole document *<br>--- | 1-5,<br>13-15<br><br><br><br>6-12 | TECHNICAL FIELDS SEARCHED (Int.Cl.7)<br>A61K |
| X<br><br><br><br><br><br>Y | MARTIN, WILLIAM H. ET AL: "Discovery of a human liver glycogen phosphorylase inhibitor that lowers blood glucose in vivo"<br>PROC. NATL. ACAD. SCI. U. S. A. (1998), 95(4), 1776-1781 , XP002122959<br>* figure 1 *<br>* discussion *<br>---<br>-/-- | 1-5,<br>13-15<br><br><br><br><br>6-12 | |

The present search report has been drawn up for all claims

| Place of search | Date of completion of the search | Examiner |
|---|---|---|
| MUNICH | 29 November 1999 | Pilling, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 99 30 6047

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br><br><br><br><br>Y | MARTIN J L ET AL: "Glucose analogue inhibitors of glycogen phosphorylase: the design of potential drugs for diabetes." BIOCHEMISTRY, (1991 OCT 22) 30 (42) 10101-16. , XP002122960<br>* abstract * | 16,17<br><br><br><br><br>6-12 | |
| X<br><br><br><br><br><br>Y | WATSON K A ET AL: "Design of inhibitors of glycogen phosphorylase: a study of alpha- and beta-C-glucosides and 1-thio-beta-D-glucose compounds." BIOCHEMISTRY, (1994 MAY 17) 33 (19) 5745-58. , XP002122961<br>* abstract * | 16,17<br><br><br><br><br><br>6-12 | |
| X<br><br><br><br><br><br><br>Y | CRUCIANI G ET AL: "Comparative molecular field analysis using GRID force-field and GOLPE variable selection methods in a study of inhibitors of glycogen phosphorylase b." JOURNAL OF MEDICINAL CHEMISTRY, (1994 AUG 5) 37 (16) 2589-601. , XP002122962<br>* abstract * | 16,17<br><br><br><br><br><br><br>6-12 | TECHNICAL FIELDS SEARCHED (Int.Cl.7) |
| X<br><br><br><br><br><br><br><br>Y | OIKONOMAKOS N G ET AL: "The design of potential antidiabetic drugs: experimental investigation of a number of beta-D-glucose analogue inhibitors of glycogen phosphorylase." EUROPEAN JOURNAL OF DRUG METABOLISM AND PHARMACOKINETICS, (1994 JUL-SEP) 19 (3) 185-92. , XP002122963<br>* abstract * | 16,17<br><br><br><br><br><br><br><br>6-12 | |

The present search report has been drawn up for all claims

| Place of search | Date of completion of the search | Examiner |
|---|---|---|
| MUNICH | 29 November 1999 | Pilling, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 30 6047

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-1999

| Patent document cited in search report | Publication date | Patent family member(s) | Publication date |
|---|---|---|---|
| WO 9639385 A | 12-12-1996 | CA 2223625 A | 12-12-1996 |
| | | AP 624 A | 19-12-1997 |
| | | AU 700887 B | 14-01-1999 |
| | | AU 5475396 A | 19-12-1996 |
| | | BG 100635 A | 30-09-1997 |
| | | BR 9602626 A | 01-09-1996 |
| | | CZ 9601627 A | 11-12-1996 |
| | | EP 0832066 A | 01-04-1998 |
| | | FI 974437 A | 05-12-1997 |
| | | HR 960266 A | 31-08-1997 |
| | | HU 9601285 A | 28-09-1998 |
| | | JP 11500445 T | 12-01-1999 |
| | | LV 11614 A | 20-12-1996 |
| | | LV 11614 B | 20-04-1997 |
| | | NO 962322 A | 09-12-1996 |
| | | PL 314603 A | 09-12-1996 |
| | | SG 45481 A | 16-01-1998 |
| | | SI 9600163 A | 28-02-1997 |
| | | SK 72096 A | 05-11-1997 |
| | | TR 970184 A | 21-03-1997 |
| WO 9639384 A | 12-12-1996 | CA 2224062 A | 12-12-1996 |
| | | AP 623 A | 19-12-1997 |
| | | AU 701465 B | 28-01-1999 |
| | | AU 5462696 A | 19-12-1996 |
| | | BG 100547 A | 31-12-1996 |
| | | BR 9602542 A | 27-10-1996 |
| | | CN 1142492 A | 12-02-1997 |
| | | CN 1140709 A | 22-01-1997 |
| | | CZ 9601573 A | 12-03-1997 |
| | | EP 0832065 A | 01-04-1998 |
| | | FI 974436 A | 27-01-1998 |
| | | HR 960244 A | 31-12-1997 |
| | | HU 9601475 A | 28-09-1998 |
| | | JP 10511687 T | 10-11-1998 |
| | | LV 11613 A | 20-12-1996 |
| | | LV 11613 B | 20-04-1997 |
| | | NO 961664 A | 09-12-1996 |
| | | NO 990405 A | 28-01-1999 |
| | | NZ 286460 A | 24-09-1998 |
| | | PL 314561 A | 09-12-1996 |
| | | SG 44947 A | 19-12-1997 |
| | | SI 9600177 A | 28-02-1997 |
| | | SK 69996 A | 06-08-1997 |
| | | TR 961048 A | 21-12-1996 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 99 30 6047

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-1999

| Patent document cited in search report | Publication date | Patent family member(s) | Publication date |
|---|---|---|---|
| EP 0846464 A | 10-06-1998 | AU 4686997 A | 11-06-1998 |
| | | CA 2223317 A | 05-06-1998 |
| | | HU 9702356 A | 28-08-1998 |
| | | JP 10194990 A | 28-07-1998 |
| | | US 5952322 A | 14-09-1999 |

EPO FORM P0459

For more details about this annex : see Official Journal of the European Patent Office, No. 12/82